# EUROPEAN PATENT APPLICATION

(11) **EP 4 660 300 A1**
(43) Date of publication of application: **10.12.2025**
(21) Application number: 24749660.7
(22) Date of filing: 29.01.2024
(51) Int. Cl.: C12N 5/10, A61K 48/00, A61P 9/10

(54) **EXOSOME, PREPARATION METHOD THEREFOR, USE THEREOF, AND PHARMACEUTICAL COMPOSITION**

(30) Priority: 30.01.2023 US 202363482264 P
(71) Applicant: Hung, Mien-Chie, Taichung City 404, Taiwan (TW)
(72) Inventor: JENG, Long-bin, Taichung City (TW); HUNG, Mien-chie, Taichung City (TW); SHYU, Woei-cherng, Taichung City (TW); LIN, Syuan-ling, Taichung City, Wuchang (TW); CHEN, Chien-lin, Taichung City, Dali (TW)
(74) Representative: Jakelski & Althoff Patentanwälte PartG mbB
(86) International application number: PCT/CN2024/074543
(87) International publication number: WO 2024/160181

(57) **Abstract**

The present disclosure provides an exosome, a preparation method of the exosome, the exosome for use, and a pharmaceutical composition including the exosome for treating an ischemia condition of a tissue. The exosome is derived from genetically engineered mesenchymal stem cells, wherein the genetically engineered mesenchymal stem cells includes an exogenous *PD-L1* gene and an exogenous *HGF* gene.

## Description

### BACKGROUND

### Technical Field

The present disclosure relates to an exosome, a preparation method, uses, and a pharmaceutical composition thereof. More particularly, the present disclosure relates to an exosome derived from a genetically engineered mesenchymal stem cell, a preparation method, uses, and a pharmaceutical composition thereof.

### Description of Related Art

An ischemia condition is a condition in which blood supply to tissues is insufficient, resulting in a lack of oxygen and nutrients. Ischemia is generally caused by vascular problems, but ischemia can also be caused by vasoconstriction, thrombosis, or embolism, leading to local anemia. In addition to lack of oxygen and nutrients, ischemia can also lead to accumulation of metabolites that can damage tissues.

When the ischemia condition occurs in the brain, it causes an ischemic stroke, which is the leading cause of death worldwide. The ischemic stroke results in rapid loss of brain function due to abnormal blood supply to the brain and predisposes to neurological behavior impairment. In general, the conventional treatment of stroke mainly relies on drugs to prevent another stroke. In addition to the opportunity to use thrombolytic agents and intracranial arterial thrombectomy in the acute stage of the ischemic stroke, other drug treatments are mainly to reduce the risk of re-stroke. However, these drugs have limited effect on the recovery of damaged brain tissue and neurological function after the stroke. More significant, the sequelae of the stroke, including paralysis, depression, disturbance of speech and visual disturbance would be remained in about 75% of its survivors. An ischemic heart disease occurs when the ischemia condition occurs in the heart. The ischemic heart disease is also the leading cause of death worldwide. Many patients with heart failure secondary to acute myocardial ischemia are not suitable for invasive treatment, and there is no effective drug therapy.

Currently in regenerative medicine, therapeutic cells such as mesenchymal stem cells have been found to be used as treatments for the ischemic heart disease and the ischemic stroke due to their pluripotency and self-renewal capacity. However, poor engraftment has been observed in the treatment of the ischemic heart disease with the therapeutic cells, possibly due to poor viability of the implanted therapeutic cells in infarcted cardiac tissue. Only 1% of viable cells are present 4 days after the transplantation, resulting in little improvement in cardiac function when the therapeutic cells are implanted into the infarct site. Therefore, there is still an urgent need for a treatment method whose efficacy or benefit can significantly improve the ischemia condition of the tissue.

### SUMMARY

According to one aspect of the present disclosure is to provide an exosome. The exosome is derived from a genetically engineered mesenchymal stem cell, and the genetically engineered mesenchymal stem cell includes an exogenous *PD-L1* gene and an exogenous *HGF* gene.

According to the exosome, wherein the exogenous *PD-L1* gene can include the amino acid sequence of SEQ ID NO: 4, and the exogenous HGF gene can include the amino acid sequence of SEQ ID NO: 5.

According to the exosome, wherein the exogenous *PD-L1* gene and the exogenous *HGF* gene can be linked by a self-cleaving peptide coding fragment.

According to the exosome, wherein the exosome can include an overexpression PD-L1 and an overexpression HGF.

According to the exosome, wherein when measured by a flow cytometry, a PD-L1 expression level on an exosome membrane of the exosome can be increased relative to a control exosome, and the control exosome is derived from a non-genetically engineered mesenchymal stem cell.

According to the exosome, wherein when measured by an ELISA, a HGF content of the exosome can be increased relative to a control exosome, and the control exosome is derived from a non-genetically engineered mesenchymal stem cell.

According to the exosome, wherein the exosome can include an overexpression CXCR4 on an exosome membrane thereof.

According to the exosome, wherein when measured by a flow cytometry, a proportion of the exosome with CXCR4 surface marker can be increased relative to a control exosome, and the control exosome is derived from a non-genetically engineered mesenchymal stem cell.

According to the exosome, wherein a particle size of the exosome can range from 30 nm to 200 nm.

According to the exosome, wherein the particle size of the exosome can range from 100 nm to 150 nm.

According to another aspect of the present disclosure is to provide a preparation method of an exosome. The preparation method of the exosome includes constructing a genetically engineered mesenchymal stem cell, performing a culture step and performing an isolation step. When constructing the genetically engineered mesenchymal stem cell, an exogenous HGF gene and an exogenous *PD-L1* gene are transferred into a mesenchymal stem cell to obtain the genetically engineered mesenchymal stem cell. In the culture step, the genetically engineered mesenchymal stem cell is cultured in a culture medium to obtain a conditioned medium. In the isolation step, an exosome is collected from the conditioned medium by an isolating method.

According to the preparation method of the exosome, wherein in the culture step, the genetically engineered mesenchymal stem cell can be cultured under a hypoxic condition.

According to the preparation method of the exosome, wherein the hypoxic condition can be an oxygen content of 3% or less.

According to the preparation method of the exosome, wherein the mesenchymal stem cell can be an adipose mesenchymal stem cell, an umbilical cord mesenchymal stem cell or a bone marrow mesenchymal stem cell.

According to the preparation method of the exosome, wherein the mesenchymal stem cell can be an adipose mesenchymal stem cell or an umbilical cord mesenchymal stem cell.

According to still another aspect of the present disclosure is to provide a pharmaceutical composition for treating an ischemia condition of a tissue. The pharmaceutical composition includes the aforementioned exosome and a pharmaceutically acceptable carrier.

According to the pharmaceutical composition, wherein an administration route of the pharmaceutical composition can be an intravenous injection, an intracarotid artery injection, an intraarterial injection or a combination thereof.

According to yet another aspect of the present disclosure is to provide the aforementioned exosome for use in treatment of an ischemia condition of a tissue.

According to the exosome for use, wherein the tissue can be a brain.

According to the exosome for use, wherein the ischemia condition can be an ischemic stroke.

According to the exosome for use, wherein the exosome reduces an area of the brain damaged by the ischemic stroke.

According to further another aspect of the present disclosure is to provide the aforementioned exosome for use in enhancing neuroregeneration or reducing neuron death.

According to still another aspect of the present disclosure is to provide the aforementioned exosome for use in reducing an inflammatory response.

### BRIEF DESCRIPTION OF THE DRAWINGS

The present disclosure can be more fully understood by reading the following detailed description of the embodiment, with reference made to the accompanying drawings as follows:
Fig. 1 is a flow diagram showing a preparation method of an exosome of the present disclosure;
Fig. 2A shows photomicrographs of differentiation of the ADSCs, the hTERT-ADSCs, and the hTERT-ADSC-PD-L1-HGF into different tissue cells;
Fig. 2B, Fig. 2C and Fig. 2D show analysis results of the PD-L1 expression level and/or the HGF content of the hTERT-ADSCs and the hTERT-ADSC-PD-L1-HGF;
Fig. 3A shows analysis results of the EXO-PD-L1-HGF production when the hTERT-ADSC-PD-L1-HGF is cultured under a normoxic condition and a hypoxic condition;
Fig. 3B shows transmission electron microscope photos of EXO and EXO-PD-L1-HGF;
Fig. 3C shows nanoparticle tracking analysis results of EXO and EXO-PD-L1-HGF;
Fig. 3D, Fig. 3E, Fig. 3F, Fig. 3G, Fig. 3H and Fig. 3I show analysis results of the HGF content, the PD-L1 expression level, the CD63 expression level, the CD9 expression level and/or the CD81 expression level of EXO and EXO-PD-L1-HGF;
Fig. 3J shows transmission electron microscope photos of UMSC-EXO and UMSC-EXO-PD-L1-HGF;
Fig. 3K shows nanoparticle tracking analysis results of UMSC-EXO and UMSC-EXO-PD-L1-HGF;
Fig. 3L, Fig. 3M and Fig. 3N show analysis results of the HGF content, the PD-L1 expression level, the CD63 expression level, the CD9 expression level and/or the CD81 expression level of UMSC-EXO and UMSC-EXO-PD-L1-HGF;
Fig. 4A shows analysis results of DiO-EXO and DiO-EXO-PD-L1-HGF uptake by primary cortical cells (PCCs);
Fig. 4B, Fig. 4C, Fig. 4D and Fig. 4E show analysis results of the TUNEL staining to detect that EXO-PD-L1-HGF reduces H₂O₂-induced apoptosis in the PCCs and the SH-SY5Y *in vitro;*
Fig. 4F and Fig. 4G show analysis results of the CCK-8 assay to detect that EXO-PD-L1-HGF reduces H₂O₂-induced cell death *in vitro;*
Fig. 4H, Fig. 4I, Fig. 4J and Fig. 4K show analysis results of the Annexin V-FITC/7-AAD double staining to detect that EXO-PD-L1-HGF reduces H₂O₂-induced apoptosis in the PCCs and the SH-SY5Y *in vitro;*
Fig. 4L shows analysis results of the Western blotting of the expression of apoptosis-related proteins in H₂O₂-induced apoptosis of the PCCs and the SH-SY5Y after the pretreatment with EXO or EXO-PD-L1-HGF;
Fig. 4M shows analysis results of the CCK-8 assay to detect that UMSC-EXO-PD-L1-HGF reduces H₂O₂-induced cell death *in vitro;*
Fig. 4N shows analysis results of the Western blotting of the expression of apoptosis-related proteins in H₂O₂-induced apoptosis of the PCCs after the pretreatment with UMSC-EXO-PD-L1-HGF;
Fig. 5A shows results of the CFSE assay to analyze that EXO-PD-L1-HGF suppresses the proliferation of T cells;
Fig. 5B shows results of the ELISA to analyze the reduction of the interferon-γ expression by EXO-PD-L1-HGF;
Fig. 5C shows analysis results of the percentage of regulatory T cells after the treatment with EXO or EXO-PD-L1-HGF;
Fig. 5D shows analysis results of stem cell marker expression of isolated neural progenitor cells (NPCs);
Fig. 5E shows analysis results of the CCK-8 assay to detect that EXO-PD-L1-HGF promotes the proliferation of the NPCs;
Fig. 5F is a heatmap of next-generation sequencing analysis of the control group, the EXO group, and the EXO-PD-L1-HGF group;
Fig. 5G shows results of Gene Set Enrichment Analysis (GSEA) of the control group, the EXO group and the EXO-PD-L1-HGF group;
Fig. 6A shows results of the Western blotting of the expression of phosphorylated STAT3 and phosphorylated FOXO3 in the PCCs and the NPCs of the PBS group, the EXO group and the EXO-PD-L1-HGF group;
Fig. 6B shows results of a human cytokine array to analyze the protein expression of cytokines in cells after the treatment with EXO or EXO-PD-L1-HGF;
Fig. 6C shows results of the ELISA to analyze the protein expression of cytokines in cells after the treatment with EXO or EXO-PD-L1-HGF;
Fig. 6D shows analysis results of the Western blotting of the expression of apoptosis-related proteins in the PCCs with the knockdown FOXO3 expression after the treatment with EXO-PD-L1-HGF;
Fig. 6E and Fig. 6F show results of the flow cytometry to analyze apoptosis in different groups;
Fig. 6G and Fig. 6H show analysis results of the number of secondary neurospheres formed by different groups of the NPCs;
Fig. 6I and Fig. 6J show analysis results of the secondary neurospheres size formed by different groups of the NPCs;
Fig. 7A and Fig. 7B show analysis results of fluorescence intensity after intravenous injection of DiD-EXO or DiD-EXO-PD-L1-HGF into middle cerebral artery occlusion (MCAO) mice;
Fig. 7C shows results of the immunofluorescence staining of EXO-PD-L1-HGF co-localized with SDF-1α at the injury site;
Fig. 7D shows analysis results of the flow cytometry to detect the CXCR4 expression level on exosome membrane of EXO and EXO-PD-L1-HGF;
Fig. 7E shows results of the immunofluorescence staining of EXO and EXO-PD-L1-HGF co-localized with GFAP⁺ cells and MAP2⁺ cells at the injury site;
Fig. 7F and Fig. 7G are results of the immunofluorescence staining showing that the knockdown FOXO3 expression suppresses the EXO-PD-L1-HGF promoted Nestin-GFP expressed NPCs migrating to the injury site;
Fig. 7H, Fig. 7I and Fig. 7J show analysis results of the treatment with EXO-PD-L1-HGF to induce the proliferation of Nestin-GFP expressed cells;
Fig. 8A, Fig. 8B, Fig. 8C and Fig. 8D show analysis results of treating EXO and EXO-PD-L1-HGF to promote the differentiation of the NPCs;
Fig. 8E shows analysis results of the MCAO mice treated with EXO-PD-L1-HGF in the rotarod test;
Fig. 8F shows analysis results of the MCAO mice treated with EXO-PD-L1-HGF in the beam walking test;
Fig. 8G and Fig. 8H show analysis results of the brain infarction in the MCAO mice treated with EXO-PD-L1-HGF;
Fig. 8I shows analysis results of the MCAO mice treated with UMSC-EXO-PD-L1-HGF in the rotarod test;
Fig. 8J shows analysis results of the MCAO mice treated with UMSC-EXO-PD-L1-HGF in the beam walking test;
Fig. 8K and Fig. 8L show analysis results of the brain infarction in the MCAO mice treated with UMSC-EXO-PD-L1-HGF;
Fig. 8M and Fig. 8N show analysis results of the TUNEL staining to detect cell apoptosis in the brain tissues of the MCAO mice treated with EXO-PD-L1-HGF;
Fig. 9A shows analysis results of the number of CD3⁺ T cells in the brain tissues of mice in different groups;
Fig. 9B, Fig. 9C, Fig. 9D, Fig. 9E and Fig. 9F show analysis results of repressing the percentage of activated dendritic cells (DCs), cytotoxic T cells, natural killer (NK) cells and M1 macrophages in the ischemic cerebral hemisphere after the treatment with EXO or EXO-PD-L1-HGF;
Fig. 9G, Fig. 9H, Fig. 9I, Fig. 9J and Fig. 9K show analysis results of repressing the percentage of activated DCs, cytotoxic T cells, NK cells and M1 macrophages in the spleen after the treatment with EXO or EXO-PD-L1-HGF;
Fig. 9L, Fig. 9M and Fig. 9N show analysis results of enhancing the percentage of regulatory B cells, regulatory T cells and M2 macrophages in the ischemic cerebral hemisphere after the treatment with EXO or EXO-PD-L1-HGF; and
Fig. 9O, Fig. 9P and Fig. 9Q show analysis results of enhancing the percentage of regulatory B cells, regulatory T cells and M2 macrophages in the spleen after the treatment with EXO or EXO-PD-L1-HGF.

### DETAILED DESCRIPTION

Unless defined otherwise, all scientific or technical terms used herein have the same meaning as those understood by persons of ordinary skill in the art to which the present disclosure belongs. Any method and material similar or equivalent to those described herein can be understood and used by those of ordinary skill in the art to practice the present disclosure.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the specification and claims of the present disclosure are approximate and can vary depending upon the desired properties sought by the present disclosure.

The term "a/an" herein should mean one or more than one of the objects described in the present disclosure. The term "and/or" means either one or both of the alternatives.

The term *"in vivo"* herein generally means inside a living organism. The term *"in vitro"* generally means outside of a living organism, such as an experiment taking place at an artificial environment created outside of the living organism.

In the specification of the present disclosure, the gene name is represented by italic letters, the symbol format of its RNA is the same as that of the gene, and the protein related to the gene is represented by non-italic letters.

The term "genetically engineered" herein means manipulating genes using genetic materials for the change of gene copies and/or gene expression level in a cell. The genetic materials can be in the form of DNA or RNA. The genetic materials can be transferred into the cell by various means including viral transduction and non-viral transfection. After being genetically engineered, the expression level of certain genes in the cell can be altered permanently or temporarily.

The term "exosome" herein, also known as "extracellular vesicles", refer to member of vesicles secreted by cells with a particle size of approximately less than 500 nm, or between 30 nm and 200 nm, which exists in various biological fluids such as amniotic fluid, urine and blood in nature. According to different cell sources, production pathways and biological characteristics, exosomes have different compositions of surface marker proteins, particle sizes, mRNA, long non-coding RNA (IncRNA) and microRNA (miRNA). In addition to natural sources, exosomes can be isolated from various cells (for example, neurons, tumor cells, kidney cells or stem cells) after culture, or can be isolated from genetically engineered cells through culture. The exosome of the present disclosure is prepared from a genetically engineered mesenchymal stem cell. In some embodiments, the particle size of "exosome" or "extracellular vesicles" of the present disclosure can range from 30 nm to 200 nm. In some embodiments, the particle size thereof can range from 100 nm to 150 nm.

The "mesenchymal stem cell" of the present disclosure can be obtained from different sources, such as bone marrow, umbilical cord blood, amniotic sac and amniotic fluid, placenta, skin, adipose, muscle, vasculature, liver, pancreas or peripheral blood. The cell source can be xenogeneic, allogeneic, or autologous. The term "mesenchymal stem cell" herein can refer to an adipose mesenchymal stem cell, an umbilical cord mesenchymal stem cell, a bone marrow mesenchymal stem cell, a dental pulp mesenchymal stem cell, an amniotic fluid mesenchymal stem cell, an amniotic mesenchymal stem cell, an induced mesenchymal stem cell (iMSC) or an immortalized mesenchymal stem cell (immortalized MSC) differentiated from induced pluripotent stem cells (iPSCs). In some embodiments, the mesenchymal stem cell can be the adipose mesenchymal stem cell. In some embodiments, the mesenchymal stem cell can be the umbilical cord mesenchymal stem cell.

The term "expression vector" herein means the agent carrying exogenous genes into the cell for expression without degradation. The expression vector in the present disclosure can be a plasmid, a viral vector, and an artificial chromosome. The exogenous genes can be located in the same expression vector or in different expression vectors.

The term "elevated expression level" herein means the increased expression level of RNA or protein of the gene of interest in the genetically engineered cell, when compared to the expression level of that gene in the non-genetically engineered cell counterpart.

The term "overexpression" herein refers to a significant increase in the expression level relative to a normal condition. For example, overexpression of a gene refers to a significant increase in the level of RNA transcribed compared to the normal condition, and increased RNA level lead to excessive accumulation of protein translated, that is, overexpression of the protein level.

In some embodiments, the term "surface marker" refers to the presence or absence of the protein on the cell surface. In some embodiments, "positive marker" means the surface marker that is present or expressed on the cell of interest and is represented by "⁺". In some embodiments, "negative marker" means the surface marker that is absent from the cell of interest and is represented by "⁻".

The terms "treatment," "treating," and "treat" herein generally refer to obtaining a desired pharmacological and/or physiological effect. The effect can be preventive in terms of completely or partially preventing a disease, a disorder, or a symptom thereof, and can be therapeutic in terms of a partial or complete cure for a disease, disorder, and/or symptoms attributed thereto. The term "treatment" used herein covers any treatment of a disease in a mammal (preferably a human) and includes suppressing development of the disease, the disorder, or the symptom thereof in a subject or relieving or ameliorating the disease, the disorder, or the symptom thereof in the subject.

The terms "individual," "subject," and "patient" herein are used interchangeably and refer to any mammalian subject for whom diagnosis, treatment, or therapy is desired.

The term "PD-L1" herein refers to programmed death-ligand 1, the 40 kDa type 1 transmembrane protein that is encoded by the CD274 gene, and PD-L1 is also known as "CD274," "B7 homolog 1," and "B7-H1." Programmed cell death protein-1 (PD-1), the receptor of PD-L1, is found on activated T cells, B cells, and myeloid cells. The binding of PD-L1 and PD-1 dampens T cell activation, decreases proliferation and cytotoxicity, and induces apoptosis. PD-L1 expressed on the mesenchymal stem cells interacts with the PD-1 to provide an inhibitory signal in regulating cellular activation and proliferation. Thus, signaling induction through PD-1 and PD-L1 pathway causes T cells exhaustion and in turn suppresses inflammatory cascade of central nervous system in murine stroke model. Moreover, the mesenchymal stem cells are found to inhibit the proliferation and function of effector T cells through both directly contacting activated T cells and indirectly secreting soluble PD-L1 in previous study. PD-L1 of the present disclosure can refer to human PD-L1 or functional variations thereof, such as functional variations whose peptide sequence is at least 95%, at least 90% or at least 80% identical to SEQ ID NO: 4 and have similar activity to PD-L1. In some embodiments, the sequence of PD-L1 is referenced as SEQ ID NO: 4.

The term "HGF" herein refers to hepatic growth factor, which is a cytokine that can significantly promote tissue repair and organ regeneration by binding to the c-Met tyrosine kinase receptor after tissue and organ damage. Previous studies pointed out the role that HGF induced functional improvement of rat's spinal cord injured (SCI) and promoted peripheral nerve regeneration in the nerve crush model. Combination of HGF and PD-L1 might act as innovative genetic candidates contributing for therapeutic intervention on neurogenesis and anti-inflammation. HGF of the present disclosure can refer to human HGF or functional variations thereof, such as functional variations whose peptide sequence is at least 95%, at least 90% or at least 80% identical to SEQ ID NO: 5 and have similar activity to HGF. In some embodiments, the sequence of HGF is referenced as SEQ ID NO: 5.

The nucleic acid for expressing PD-L1 and HGF can be a polycistronic construct, which allows the expression of two exogenous genes (that is the *PD-L1* gene and the *HGF* gene) under the control of a single promoter or two different promoters. The two exogenous genes can be expressed by the same promoter, encoded in the same protein sequence and separated by a self-cleaving peptide, or the two exogenous genes can be expressed in two different gene vectors. In some embodiments, the exogenous genes (that is the *PD-L1* gene and the *HGF* gene) are linked by a self-cleaving peptide coding fragment. The self-cleaving peptide encoded by the self-cleaving peptide coding fragment used in the nucleic acid construct of the present disclosure can be a F2A peptide derived from Foot-and-mouth disease virus (FMDV), a P2A peptide derived from Porcine teschovirus (PTV), a T2A peptide derived from Thosea asigna virus (TaV) and/or a E2A peptide derived from Equine rhinitis A virus (ERAV). In some embodiments, the self-cleaving peptide coding fragment is a T2A coding fragment having the sequence referenced as SEQ ID NO: 3, which encodes the T2A peptide having the sequence referenced as SEQ ID NO: 6.

The term "CXCR4" refer to CXC motif chemokine receptor 4, which is a receptor specific for stromal cell-derived factor-1α (SDF-1α). The CXCR4 is expressed on most body tissues and organs. The CXCR4 is a G protein-coupled receptor (GPCR) composed of 352 amino acids, and has seven transmembrane structures. SDF-1α, the ligand of CXCR4, endows with a potent chemotactic activity for lymphocytes.

In some embodiments, a PD-L1 expression level on an exosome membrane of the exosome derived from the genetically engineered mesenchymal stem cell is increased relative to a control exosome derived from a non-genetically engineered mesenchymal stem cell, as measured by a flow cytometry or a Western blotting. When measured by the flow cytometry, the number of the exosomes with high PD-L1 expression level is increased by 20%, 30%, 40%, 50%, 60%, 70%, 80%, 90% or more. In some embodiments, the number of the exosomes with high PD-L1 expression level is increased by about 50%. In some embodiments, the number of the exosomes with high PD-L1 expression level is increased by about 90%. A proportion of the PD-L1 surface markers in a therapeutic exosome can be 95% or more, 90% or more, 80% or more, 70% or more, 60% or more, or 50% or more. In some embodiments, the proportion of the PD-L1 surface markers in the therapeutic exosome is 90% or more.

In some embodiments, a HGF content of the exosome derived from the genetically engineered mesenchymal stem cell is significantly increased relative to the control exosome derived from the non-genetically engineered mesenchymal stem cell, as measured by an enzyme-linked immunosorbent assay (ELISA) or the Western blotting. When measured by the ELISA, the HGF content of the exosome is increased by approximately 25%, 50%, 75%, 100%, 150%, 200%, or 200% or more. In some embodiments, the HGF content of the exosome is increased by about 200%. In some embodiments, the HGF content of the exosome is increased by about 50%.

In some embodiments, a CXCR4 expression level on the exosome membrane of the exosome derived from the genetically engineered mesenchymal stem cell is increased relative to the control exosome derived from the non-genetically engineered mesenchymal stem cell, as measured by the flow cytometry or the Western blotting. When measured by the flow cytometry, a proportion of the exosomes with the CXCR4 surface markers is increased by 5% to 10%, 5% to 15%, 5% to 20%, 10% to 20%, 20% to 30%, 30% to 40%, 40% to 50%, 10% to 30%, 20% to 40%, 30% to 50%, or is increased by at least 5% or more, at least 10% or more, at least 20% or more, at least 30% or more, at least 40% or more, or at least 50% or more relative to that of the control exosomes. In some embodiments, the proportion of the exosomes with the CXCR4 surface markers is increased by 5% to 10%, or at least 5% or more relative to that of the control exosomes. The proportion of the CXCR4 surface markers in the therapeutic exosome can be 5% or more, 10% or more, 15% or more, 20% or more, 30% or more, or 40% or more. In some embodiments, the proportion of the CXCR4 surface markers in therapeutic exosome can be 5% or more.

Augmented proliferation of endogenous neural progenitor cells (NPCs) at the subventricular zone (SVZ) neurogenic niches provided a significant form of neuroplasticity. During stroke induction, a significant population of the NPCs within the SVZ diverted from their initial migration route and navigated toward the peri-infarct region, signifying a possible role for SVZ-derived NPCs in post stroke recovery. Previous evidence demonstrated that FOXO3 modulated the NPCs homeostasis to preserve cells quiescence and prevent premature differentiation by inducing a program of self-renewal genes. Upregulation of FOXO3 inducing by hypoxia-ischemia linked to the hypothesis that turning on FOXO3 expression by specific therapeutic strategy is capable of promoting the proliferation of the NPCs for stroke recovery.

In order to enhance the treatment efficacy, the genetically engineered mesenchymal stem cell obtained by transferring the exogenous *PD-L1* gene and the exogenous HGF gene into the mesenchymal stem cell and further collecting the exosome derived from the genetically engineered mesenchymal stem cell might be a better strategy for rescuing the injured neuroglial tissue after the stroke. Therefore, the present disclosure provides the exosome derived from the genetically engineered mesenchymal stem cell, which includes an overexpression PD-L1 and an overexpression HGF. In addition, the exosome of the present disclosure includes an overexpression of CXCR4 on the exosome membrane thereof. In the specification of the present disclosure, *in vitro* experiments and animal experiments are used to confirm the therapeutic potential of the exosome of the present disclosure in promoting neuroprotection, anti-inflammation and neurogenesis after the ischemic stroke, indicating that the exosome of the present disclosure has the ability to enhance neuroregeneration or reduce neuron death, and can induce enhanced neural recovery and reduced infarct volume in an ischemic stroke mouse model. Through exploring the molecular mechanism of STAT3-FOXO3 signaling axis, the exosome of the present disclosure can be used as a new cell-free therapeutic product that can be able to augment neurological functional recovery after the ischemic stroke.

The genetically engineered mesenchymal stem cells according to the present disclosure are modified to express PD-L1 and HGF. As used herein, the term "modified to express" refers to transferring an exogenous gene or a gene fragment into the mesenchymal stem cell so that it can express the exogenous gene or the gene fragment. Preferably, the modification does not alter the differentiation potential of the mesenchymal stem cell, nor does it alter the immunomodulatory properties of the mesenchymal stem cell. In another aspect, the modification is preferred to be a stable modification, and the expression can be persistent or inducible. The mesenchymal stem cell according to the present disclosure are modified to express PD-L1 and HGF and still have pluripotent differentiation potential, such as, but not limited to, adipogenesis, chondrogenesis, osteogenesis and vascularization, that is similar with the non-genetically engineered mesenchymal stem cell counterpart.

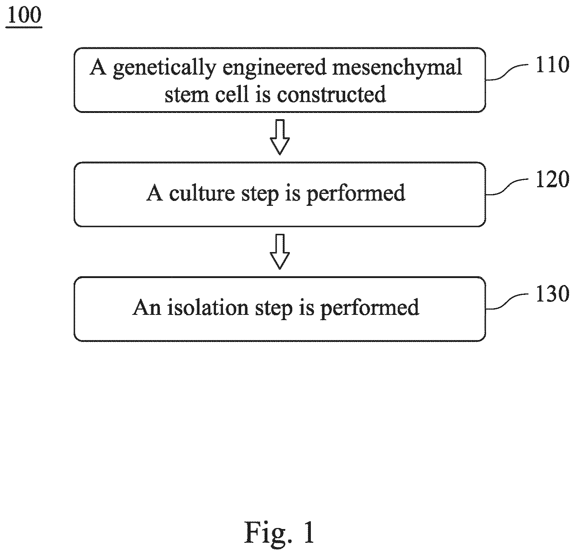

The present disclosure also provides a preparation method of an exosome 100. Reference is made to Fig. 1, which is a flow diagram showing the preparation method of the exosome 100 of the present disclosure including Step 110, Step 120 and Step 130.

In Step 110, a genetically engineered mesenchymal stem cell is constructed. An exogenous *HGF* gene and an exogenous *PD-L1* gene are transferred into a mesenchymal stem cell to obtain the genetically engineered mesenchymal stem cell. The mesenchymal stem cell can be obtained from different sources, preferably from an adipose tissue, an umbilical cord or a bone marrow. Depending on the source, the mesenchymal stem cell can be an adipose mesenchymal stem cell (ADSC), an umbilical cord mesenchymal stem cell (UMSC) or a bone marrow mesenchymal stem cell (BMSC).

The term "transferred" mean transferring the genetic material into cells through various methods, including viral transduction and non-viral transfection. The transduction means using a virus to deliver the genetic material into cells, wherein the virus can be an integrating or non-integrating virus. The integrating virus used in the present disclosure can be lentivirus or retrovirus. The integrating virus allows integration of its encoding genes into the transduced cells that are infected with the viral particles. The non-integrating virus can be adenovirus or Sendai virus. Non-viral methods can also be used in the present disclosure such as by transfecting DNA material or RNA material into the cells. The DNA material can be in the form of PiggyBac, minicircle vector, or episomal plasmid. The RNA material can be in the form of mRNA or miRNA.

The genetically engineered mesenchymal stem cell includes an expression vector, and the expression vector includes the exogenous *PD-L1* gene and the exogenous *HGF* gene. In addition to the exogenous *PD-L1* gene and the exogenous *HGF* gene, the expression vector can include one or more control sequences to regulate the expression of the polynucleotide of the present disclosure. Manipulation of the isolated polynucleotide prior to its insertion into a vector can be desirable or necessary depending on the expression vector utilized. Techniques for modifying polynucleotides and nucleic acid sequences utilizing recombinant DNA methods are well known in the art. In some embodiments, the control sequences include promoters, leader sequences, polyadenylation sequences, propeptide sequences, signal peptide sequences, and transcription terminators. In some embodiments, suitable promoters are selected based on host cell selection. In some embodiments, the exogenous *PD-L1* gene and the exogenous *HGF* gene are present on the same expression vector. In some embodiments, the exogenous *PD-L1* gene and the exogenous *HGF* gene are present on different expression vectors.

In Step 120, a culture step is performed. The genetically engineered mesenchymal stem cell is cultured in a culture medium to obtain a conditioned medium. The conditioned medium is a supernatant of cell culture. In addition, the genetically engineered mesenchymal stem cell can be cultured under a hypoxic condition. The hypoxic conditions can be adjusted according to the cell culture process. Specifically, the hypoxic condition can be an oxygen content of 10% or less, 5% or less, 1% to 5%, 2% to 10%, 2% to 8%, 2% to 5% or 2% to 4%. In some embodiments, the genetically engineered mesenchymal stem cell of the present disclosure can be cultured under the hypoxic condition with the oxygen content of 3%.

In Step 130, an isolation step is performed. An exosome is collected from the conditioned medium by an isolating method. The isolating method can include conventional exosome isolation methods such as ultrahigh-speed centrifugation, polymer precipitation, size exclusion chromatography, and tangential flow filtration (TFF).

The present disclosure also provides a pharmaceutical composition for treating an ischemia condition of a tissue. The pharmaceutical composition includes the aforementioned exosome and a pharmaceutically acceptable carrier.

The pharmaceutical composition of the present disclosure includes an effective amount of the exosomes to treat the ischemia condition of the tissue. The pharmaceutical composition of the present disclosure can be in liquid form or lyophilized form, which can include pharmaceutically acceptable excipients that stabilize the pharmaceutical composition of the present disclosure. For administration to mammals, the pharmaceutical composition of the present disclosure can suspend the exosomes in a pharmaceutically acceptable carrier, such as buffered saline and other pharmaceutically acceptable saline solutions.

An administration route of the pharmaceutical composition including the exosome of the present disclosure depends on a tissue or an organ in need of treatment. In some embodiments in the subject suffering from myocardial infarction, the administration route of the exosome can be intravenous injection, carotid artery injection, arterial injection, or a combination thereof. In the subject with the stroke or acute myocardial infarction (AMI), the administration route can be a combination of carotid artery injection and intravenous injection. In addition, the administration route also includes injecting the exosome of the present disclosure into a subject in need of such treatment through an intraarterial route in combination with an intravenous route. Preferably, the intraarterial route is through the carotid artery.

The present disclosure also provides a use of the aforementioned exosome for preparing drugs for treating the ischemia condition of a tissue. The term "ischemia condition" refers to a condition resulting from or accompanied by an ischemic disease, which is usually characterized as reduced blood flow to a tissue or an organ due to undesirable vascular condition, such as blood vessel stenosis or aneurysm rupture. The myocardial infarction (MI), the ischemic stroke and critical limb ischemia are the three most common ischemic diseases. The tissue can be a brain and a heart. When the tissue is the brain, the ischemia condition can be the ischemic stroke. When the tissue is the heart, the ischemia condition can be the MI.

The ischemic disease includes, but is not limited to, the ischemic heart disease, myocardial ischemia, the stroke, the MI, ischemic renal disease (IRD), renal failure (such as acute renal failure). Preferably, the MI is the AMI. The administration of the present disclosure also includes reducing inflammation in the ischemic tissue.

The present disclosure also provides the use of the aforementioned exosome, which are used to prepare drugs that enhance neuroregeneration or reduce neuron death. In addition, the exosome of the present disclosure can also be used to prepare drugs that reduce an inflammatory response.

The exosome of the present disclosure can be administered with an additional active agent. For example, the exosome and the additional active agent can be administered concurrently, separately or simultaneously, or the exosome and the additional active agent can be administered periodically.

It is to be understood that if any prior art publication is referred to herein; such reference does not constitute an admission that the publication forms a part of the common general knowledge in the art.

The following specific examples are hereby used to further illustrate the present disclosure, so that those skilled in the art can fully utilize and practice the present disclosure without over-interpretation. These test examples should not be regarded as limiting the scope of the present disclosure, but are used to illustrate the materials and methods of practicing the present disclosure.

### I. The exosome of the present disclosure

### 1.1 Constructing the genetically engineered mesenchymal stem cell of the present disclosure

### 1.1.1 The genetically engineered mesenchymal stem cell derived from the adipose tissue

The genetically engineered mesenchymal stem cell of the present disclosure is first constructed. The adipose tissue-derived mesenchymal stem cells used are human telomerase reverse transcriptase immortalized adipose tissue derived MSCs cell line (hereinafter referred to as hTERT-ADSCs), were purchased from ATCC (CRL-2266), who provided the certificate of analysis (COA) of the hTERT-ADSCs and showed the immortalized cell still has the characteristics of MSCs including high expression of CD73, CD90 and CD105, while lacking expression of CD45, CD34, CD19, CD11b and HLA-DR. The hTERT-ADSCs are maintained in MSC NutriStem^{®} XF Medium (Biological Industries) with Supplement Mix, and grown in a humidified atmosphere of 5% CO₂ at 37°C. Moreover, the hTERT-ADSCs are able to differentiate into osteoblasts, chondrocytes and adipocytes *in vitro.*

In addition, adipose stem cells (ADSCs) are used as a control group. The collected human adipose tissues approved by the Institutional Review Board (IRB) were washed three times with Ca²⁺ and Mg²⁺-free PBS (DPBS, Life Technology), and then were mechanically cut by scissors to dissociate adipose mesenchymal stem cells from the adipose tissue. The adipose tissue was then extensively cut into pieces smaller than 0.5 cm³ and treated with collagenase type 1 (Sigma-Aldrich). The explants then were cultured in MSC NutriStem^{®} XF Medium with Supplement Mix (Biological Industries) and antibiotics at 37°C in a 5% CO₂ humidified atmosphere, and were left undisturbed for 5-7 days to allow for migration of the ADSCs from the explants. The cellular morphology of the ADSCs became homogenously spindle shaped in cultures after 4-8 passages, and the specific surface molecules of the ADSCs were characterized by flow cytometric analysis. The cells were detached with TrypLE^{™} Select Enzyme (Gibco), washed with PBS and incubated with the respective antibodies conjugated with fluorescein isothiocyanate (FITC) or phycoerythrin (PE) including CD13, CD29, CD44, CD73, CD90, CD105, CD166, CD49b, CD1q, CD3, CD10, CD14, CD31, CD34, CD45, CD49d, CD56, CD117, HLA-ABC, and HLA-DR (BD Biosciences). Thereafter, the cells were analyzed using a Becton Dickinson flow cytometer and the FlowJo v.7.6 software.

A PiggyBac transposon system was used to establish the genetically engineered mesenchymal stem cell of the present disclosure. PD-L1 (NM_014143) human-tagged ORF clone (Cat. No. RC213071, OriGene Technologies) was used as a PCR template for PD-L1 cDNA, and the sequence of the obtained PD-L1 cDNA is referenced as SEQ ID NO: 1. HGF (NM_000601) human-tagged ORF clone (Cat. No. RC215593L3, OriGene Technologies) was used as a PCR template for HGF cDNA, and the sequence of the obtained HGF cDNA is referenced as SEQ ID NO: 2. A bi-cistronic expression construct was amplified by specific restriction enzyme linkers and PCR to generate a PD-L1-T2A-HGF fragment (sequence is referenced as SEQ ID NO: 7), in which the sequence of the T2A coding fragment is referenced as SEQ ID NO: 3. Subsequently, the PD-L1-T2A-HGF fragment was sub-cloned into the PB-CMV-MCS-EF1α-Puro PiggyBac cDNA cloning and expression vector (Cat. No. PB510B-1, System Biosciences) by Notl to construct a PB510B-PD-L1-HGF expression construct (hereinafter referred to as PB510B-PD-L1-HGF). The pPB-CMV-MCS-EF1α-Puro includes a multiple cloning site (MCS) driven by human EF1α, PiggyBac terminal repeats (PB-TRs), core insulators (CIs) and a puromycin selection marker.

To obtain stable genetically engineered mesenchymal stem cell, the above PB510B-PD-L1-HGF was co-transfected with the PiggyBac transposase (System Biosciences) into the hTERT-ADSCs by Amaxa Nucleofector^{™} 2b device (Lonza), and stable adipose tissue-derived genetically engineered mesenchymal stem cells (hereinafter referred to as hTERT-ADSC-PD-L1-HGF) were selected by puromycin.

First, in order to verify that the genetic manipulation will not disturb the stemness of the genetically engineered mesenchymal stem cell, the ADSCs, the hTERT-ADSCs and the hTERT-ADSC-PD-L1-HGF were tested for *in vitro* differentiation assay including adipogenesis, chondrogenesis and osteogenesis.

In the *in vitro* differentiation assay for adipogenesis, confluent monolayer cultures of the ADSCs, the hTERT-ADSCs and the hTERT-ADSC-PD-L1-HGF were grown in adipogenic differentiation medium, consisting of StemPro adipogenesis differentiation basal medium (A10410-01, Gibco), supplement (A10065-01, Gibco), 15% rabbit serum and 100 mg/mL penicillin and streptomycin (Sigma-Aldrich). Cells maintained in ordinary MSC NutriStem^{®} XF medium were served as a negative control. The adipogenic medium was changed three times per week until 7-14 days. To assess adipogenesis, the cells in each group were stained with 0.3% oil red staining solution (Sigma-Aldrich) for 30 minutes at room temperature (RT) and preventing from light and to label intracellular lipid accumulation.

In the *in vitro* differentiation assay for chondrogenesis, cultures of the ADSC, the hTERT-ADSCs and the hTERT-ADSC-PD-L1-HGF were induced using a high-density (2×10⁶/100 µL) cell suspended culture system. Cells were washed in chondrogenic differentiation medium consisting of osteocyte/chondrocyte differentiation basal medium (A10069-01, Gibco), supplement (A10064-01), and 100 mg/mL penicillin and streptomycin (Sigma-Aldrich). The chondrogenic differentiation medium was changed twice per week until 14-21 days. Chondrogenesis in pellet cultureswere confirmed histologically using Alcian blue staining (Sigma-Aldrich) of sulfated proteoglycans for overnight at RT and preventing from light.

In the *in vitro* differentiation assay for osteogenesis, confluent monolayer of the ADSCs, the hTERT-ADSCs and the hTERT-ADSC-PD-L1-HGF cultures were grown in osteogenic differentiation medium consisting of osteocyte/chondrocyte differentiation basal medium (A10069-01, Gibco), supplement (A10066-01), and 100 mg/mL penicillin and streptomycin (Sigma-Aldrich). Cells maintained in ordinary MSC NutriStem^{®} XF medium were served as negative control. The osteogenic medium was changed three times per week until 21-28 days. Levels of osteogenesis were determined using 2% Alizarin red S staining solution (Sigma-Aldrich) for 45 minutes at RT and preventing from light and to detect calcium mineralization.

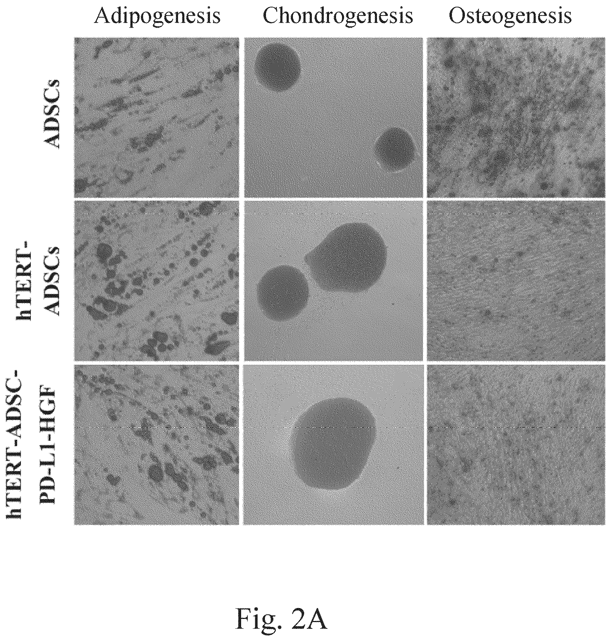

Reference is made to Fig. 2A, which shows photomicrographs of differentiation of the ADSCs, the hTERT-ADSCs, and the hTERT-ADSC-PD-L1-HGF into different tissue cells. The results in Fig. 2A show that the differentiation potential of the hTERT-ADSC-PD-L1-HGF into osteoblasts, chondrocytes and adipocytes is the same as the parent hTERT-ADSCs and primary ADSCs.

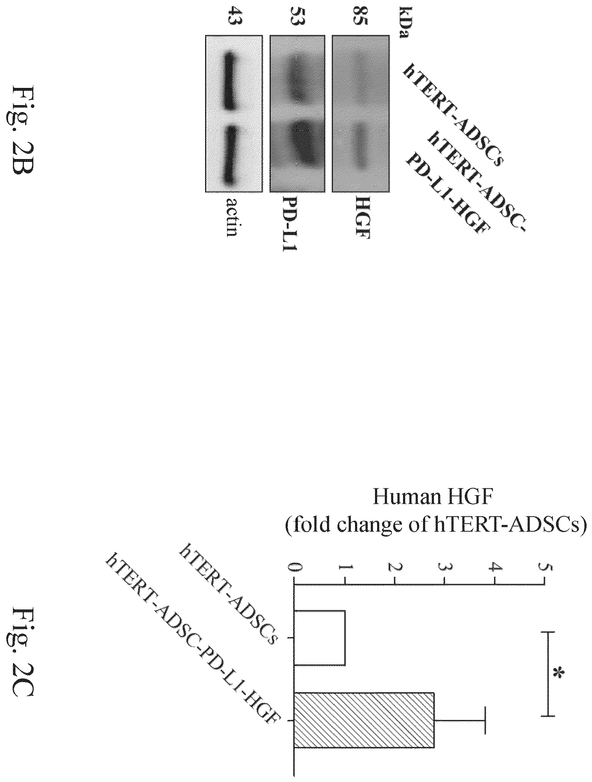

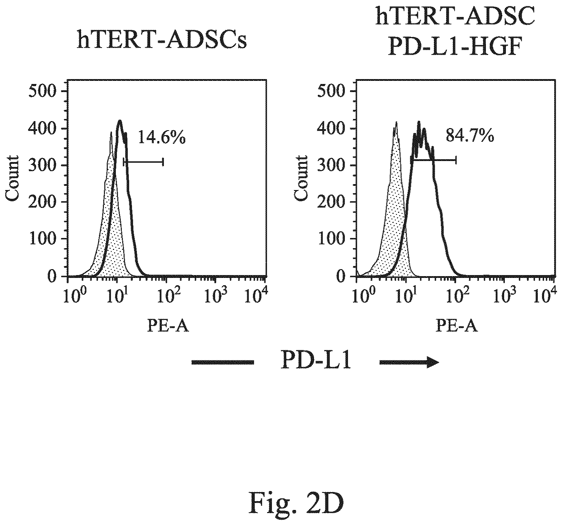

To validate whether the PB510B-PD-L1-HGF was successfully electroporated into the hTERT-ADSCs, the PD-L1 expression level and/or the HGF content in the hTERT-ADSCs and the hTERT-ADSC-PD-L1-HGF were analyzed by the Western blotting, the ELISA and the flow cytometry. The primary antibodies used were PD-L1 (OriGene), HGF (Millipore), and actin (Millipore), wherein actin was an internal control. Reference is made to Fig. 2B to Fig. 2D, which show analysis results of the PD-L1 expression level and/or the HGF content of the hTERT-ADSCs and the hTERT-ADSC-PD-L1-HGF. Fig. 2B shows analysis results of the Western blotting, Fig. 2C shows analysis results of the ELISA, and Fig. 2D shows analysis results of the flow cytometry. The results in Fig. 2B show that the protein level of PD-L1 and the protein level of HGF were significantly overexpressed in the hTERT-ADSC-PD-L1-HGF compared with that of the hTERT-ADSCs. The results in Fig. 2C show that the HGF content was approximately increased 1.5-folds in the hTERT-ADSC-PD-L1-HGF compared with that of the hTERT-ADSCs. Moreover, the results in Fig. 2D show that the PD-L1 expression level on cell surface was also upregulated in the hTERT-ADSC-PD-L1-HGF (approximately 85%) which are compared with hTERT-ADSCs.

### 1.1.2 The genetically engineered mesenchymal stem cell derived from the umbilical cord

In this test example, the mesenchymal stem cell derived from umbilical cord is used to construct the genetically engineered mesenchymal stem cell of the present disclosure. The collected human umbilical cord tissues approved by the Institutional Review Board (IRB) were washed three times with Ca²⁺ and Mg²⁺ free PBS (DPBS, Life Technology), then were mechanically cut by scissors in a midline direction, and the vessels of the umbilical artery, vein, and outlining membrane were dissociated from Wharton's jelly (WJ). The WJ content was then extensively cut into pieces smaller than 0.5 cm³, treated with collagenase type 1 (Sigma-Aldrich), and incubated for 3 hours in 5% CO₂ at 37°C. The explants then were cultured in MSC NutriStem^{®} XF Medium with NutriStem^{®} Supplement Mix (Sartorius), 5% UltraGRO^{™}-Advanced-PURE Cell Culture Supplement (AventaCell Biomedical) and antibiotics in 5% CO₂ at 37°C, and were left undisturbed for 5-7 days to allow migration of the umbilical cord mesenchymal stem cells (UMSCs) from the explants. The cellular morphology of the UMSCs became homogenously spindle-shaped in cultures after 4-8 passages. The specific surface molecules of the UMSCs were characterized by the flow cytometry. The cells were detached with TrypLE^{™} Select Enzyme (Gibco), washed with PBS, and incubated with the respective antibodies conjugated with fluorescein isothiocyanate (FITC) or phycoerythrin (PE) including CD13, CD29, CD44, CD73, CD90, CD105, CD166, CD49b, CD1d, CD3, CD10, CD14, CD31, CD34, CD45, CD49d, CD56, CD117, HLA-ABC, and HLA-DR (BD Biosciences). Subsequently, the cells were analyzed using a Becton Dickinson flow cytometer and the FlowJo v.7.6 software.

To generate stable genetically engineered mesenchymal stem cell, the above PB510B-PD-L1-HGF was co-transfected with the PiggyBac transposase (System Biosciences) into the UMSCs by Amaxa Nucleofector^{™} 2b device (Lonza), and stable umbilical cord-derived genetically engineered mesenchymal stem cells (hereinafter referred to as UMSC-PD-L1-HGF) were selected by puromycin.

### 1.2 Preparation and isolation of the exosome derived from the genetically engineered mesenchymal stem cell

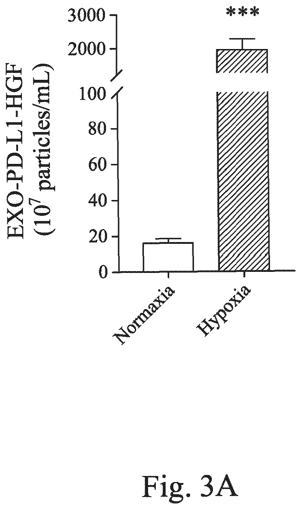

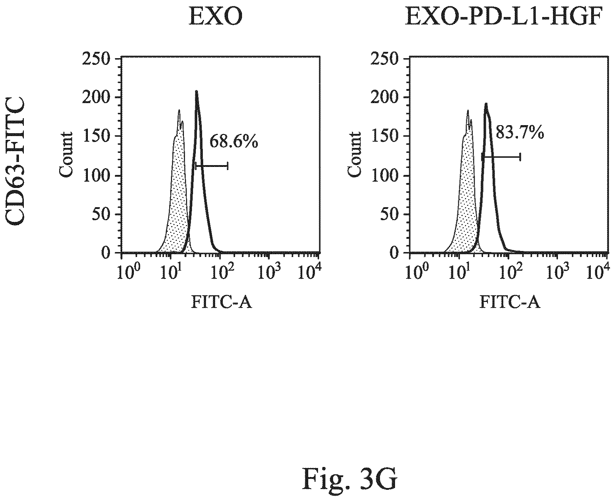

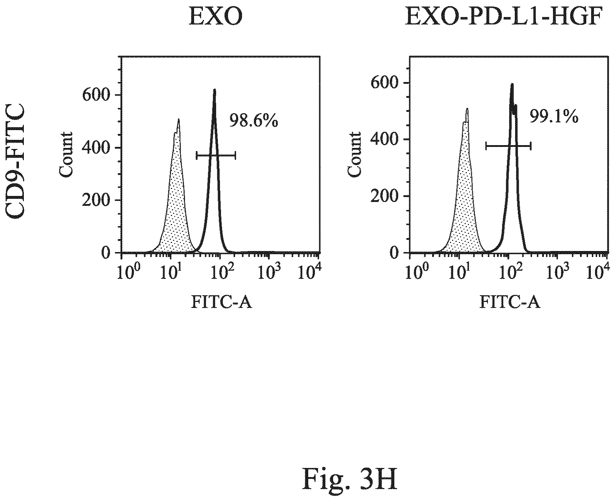

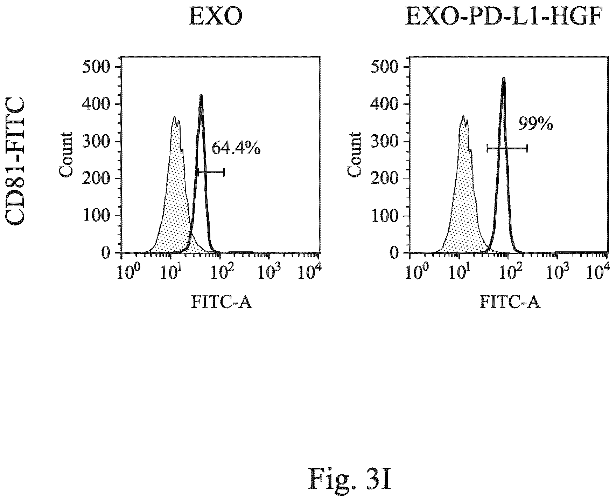

In order to confirm the optimal condition for the production of the exosome, the hTERT-ADSC-PD-L1-HGF was first cultured under a normoxic condition (20% O₂) or a hypoxic condition (3% O₂) for 72 hours. Reference is made to Fig. 3A, which shows analysis results of the EXO-PD-L1-HGF production when the hTERT-ADSC-PD-L1-HGF is cultured under the normoxic condition and the hypoxic condition. The results in Fig. 3A show that the exosome (EXO-PD-L1-HGF) production under the hypoxic condition (3% O₂) is higher than that under the normoxic condition. Therefore, the exosome of the present disclosure was prepared under the hypoxic condition in subsequent experiments.

Further, the hTERT-ADSC-PD-L1-HGF was used to prepare the exosome of the present disclosure (hereinafter referred to as EXO-PD-L1-HGF), and the hTERT-ADSCs was used to prepare the control exosome (hereinafter referred to as EXO). The hTERT-ADSCs and the hTERT-ADSC-PD-L1-HGF were cultured in conventional culture medium (MSC NutriStem^{®} XF medium with penicillin-streptomycin) on 75 cm² tissue culture flasks. To prepare EXO and EXO-PD-L1-HGF, the conventional culture medium for the hTERT-ADSCs and the hTERT-ADSC-PD-L1-HGF was replaced with fresh DMEM medium without serum and incubated under the hypoxic condition (3% O₂), and a conditioned medium can be obtained after 72 hours of culture. The conditioned medium was then collected, centrifuged at 500×g for 10 minutes, and centrifuged again at 3,000×g for a further 20 minutes to remove cells and debris. The isolation step was then performed by the polymer precipitation or the tangential flow filtration. Based on protocol for the polymer precipitation, the centrifugal supernatant was centrifuged using an Amicon ultra-15 centrifugal filter 3K device (Millipore) at 5,000×g for 60 minutes at 4°C, and the centrifuged supernatant was filtered using a 0.22 µm pore filter to obtain a concentrated supernatant. The concentrated supernatant was mixed with Total Exosome Isolation reagent (Invitrogen) and incubated at 4°C overnight, and then centrifuged at 10,000×g for 1 hour at 4°C to collect EXO and EXO-PD-L1-HGF. Based on protocol for the tangential flow filtration, the centrifuged supernatant was subjected to tangential flow filtration using a hollow fiber filter with a 300 kDa molecular weight cutoff (MWCO) and concentrated 10-fold. The final product was sterile filtered using the 0.22 µm pore filter to collect EXO and EXO-PD-L1-HGF. The obtained exosomes were used freshly or stored at -80°C after resuspension in sterile 1×PBS or RIPA buffer. For storage at -80°C, the exosomes were mixed with a lyophilized protective agent and frozen at -80°C for 6-10 hours at atmospheric pressure, and dried 21 hours at -50°C in a vacuum. The obtained lyophilized exosomes can be storage at -80°C.

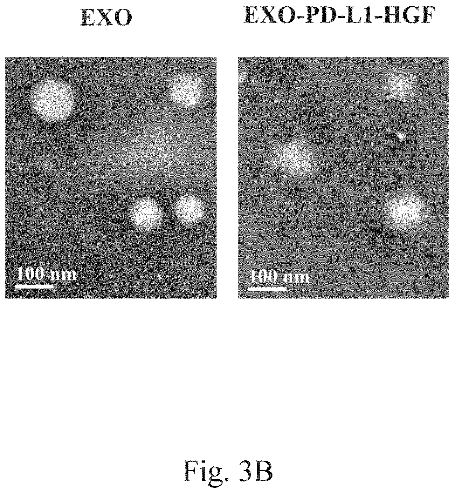

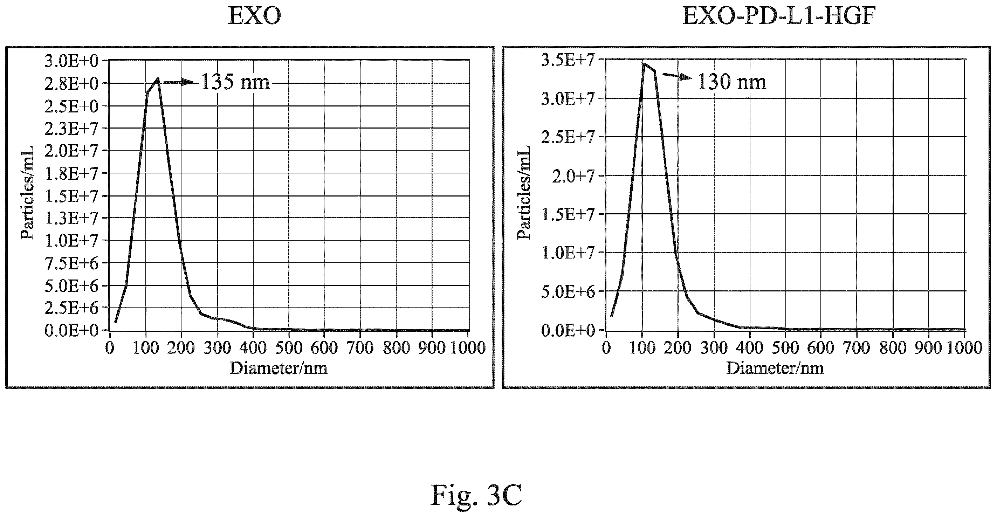

The morphology and particle size of EXO and EXO-PD-L1-HGF were analyzed by transmission electron microscopy (TEM) and nanoparticle tracking analysis (NTA). Reference is made to Fig. 3B and Fig. 3C. Fig. 3B shows transmission electron microscope photos of EXO and EXO-PD-L1-HGF, and Fig. 3C shows nanoparticle tracking analysis results of EXO and EXO-PD-L1-HGF. The results in Fig. 3B show that the morphology of EXO and EXO-PD-L1-HGF is a homogenous round-shaped membrane vesicle. The results in Fig. 3C show that the particle size distribution of EXO and EXO-PD-L1-HGF is between 30 nm to 200 nm, and is mainly distributed between 100 nm and 150 nm.

The HGF content, the PD-L1 expression level, the CD63 expression level, the CD9 expression level and/or the CD81 expression level of EXO and EXO-PD-L1-HGF were analyzed by the Western blotting, the ELISA and the flow cytometry. The primary antibodies used were PD-L1 (OriGene), HGF (Millipore), CD63 (Invitrogen), CD9 (ABclonal) and CD81 (ABclonal).

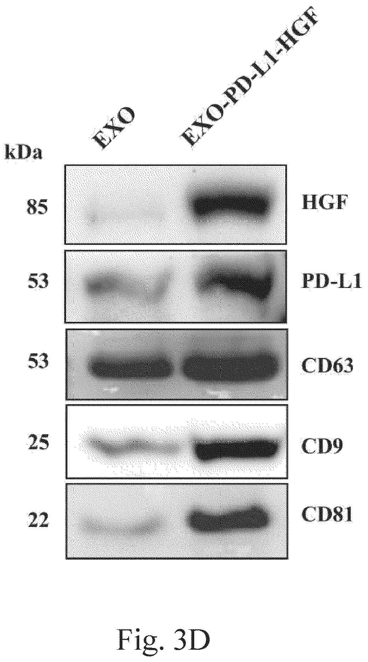

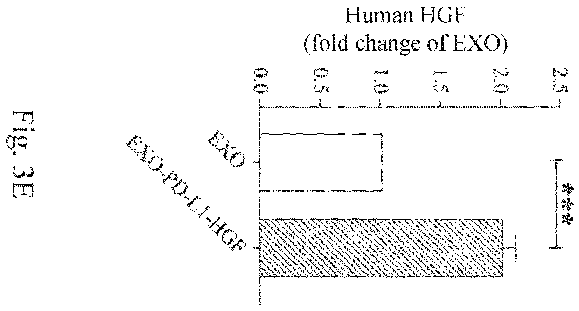

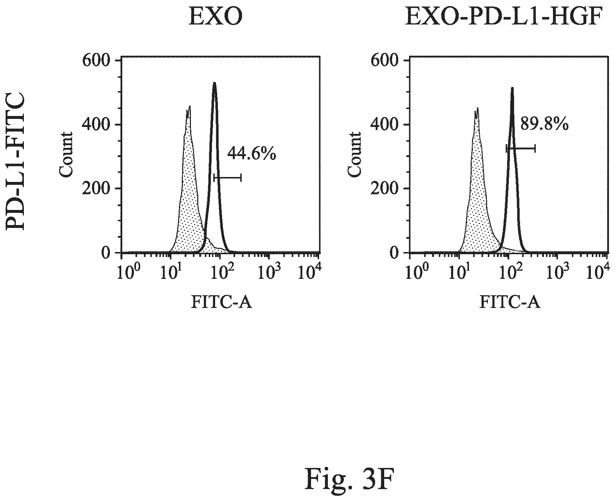

Reference is made to Fig. 3D to Fig. 3I, which show analysis results of the HGF content, the PD-L1 expression level, the CD63 expression level, the CD9 expression level and/or the CD81 expression level of EXO and EXO-PD-L1-HGF. Fig. 3D shows analysis results of the Western blotting, Fig. 3E shows analysis results of the ELISA, and Fig. 3F to Fig. 3I show analysis results of the flow cytometry of PD-L1, CD63, CD9 and CD81 respectively. The results in Fig. 3D show that both EXO and EXO-PD-L1-HGF express specific exosomal markers CD9, CD63 and CD81, confirming the identity of the exosome. Moreover, the PD-L1 expression level and the HGF content in EXO-PD-L1-HGF are more than that in EXO. The results in Fig. 3E show that the HGF content in EXO-PD-L1-HGF is significantly higher than that in EXO, which are consistent with the results in Fig. 3D. The results in Fig. 3F to Fig. 3I show that the PD-L1 expression level, the CD9 expression level, the CD63 expression level and the CD81 expression level in EXO-PD-L1-HGF are significantly higher than that in EXO, which are also consistent with the results in Fig. 3D.

In addition, UMSC-PD-L1-HGF was used to prepare the exosome of the present disclosure (hereinafter referred to as UMSC-EXO-PD-L1-HGF), and the UMSCs was used to prepare the control exosome (hereinafter referred to as UMSC-EXO). The UMSCs and the UMSC-PD-L1-HGF were cultured in conventional culture medium (MSC NutriStem^{®} XF medium with penicillin-streptomycin) on 75 cm² tissue culture flasks. To prepare UMSC-EXO and UMSC-EXO-PD-L1-HGF, the conventional culture medium for the UMSCs and the UMSC-PD-L1-HGF was replaced with fresh DMEM medium without serum and incubated under the hypoxic condition (3% O₂), and a conditioned medium can be obtained after 72 hours of culture. The conditioned medium was then collected, centrifuged at 500×g for 10 minutes, and centrifuged again at 3,000×g for a further 20 minutes to remove cells and debris. The isolation step was then performed by the polymer precipitation or the tangential flow filtration to collect UMSC-EXO and UMSC-EXO-PD-L1-HGF.

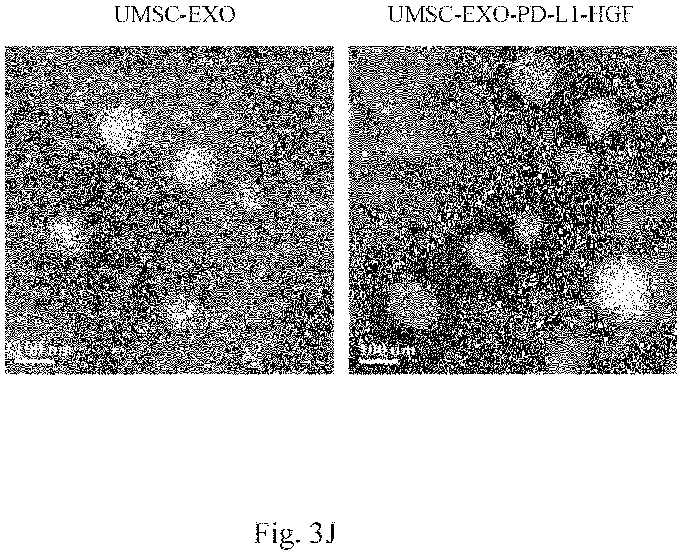

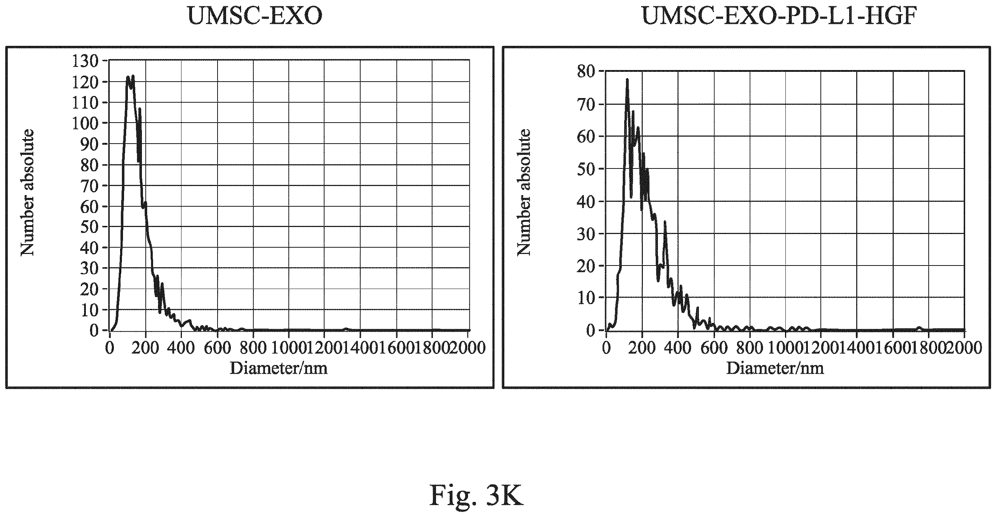

The morphology and particle size of UMSC-EXO and UMSC-EXO-PD-L1-HGF were analyzed by TEM and NTA. Reference is made to Fig. 3J and Fig. 3K, which show TEM photos and NTA results of UMSC-EXO and UMSC-EXO-PD-L1-HGF respectively. The results in Fig. 3J show that the morphology of UMSC-EXO and UMSC-EXO-PD-L1-HGF is the homogenous round-shaped membrane vesicle. The results in Fig. 3K show that the particle size distribution of UMSC-EXO and UMSC-EXO-PD-L1-HGF is between 30 nm to 200 nm, and is mainly distributed between 100 nm and 150 nm. In Fig. 3K, the number of UMSC-EXO is approximately 9.3×10¹¹ particles/mL, and the number of UMSC-EXO-PD-L1-HGF is approximately 1.5×10¹² particles/mL.

The HGF content, the PD-L1 expression level, the CD63 expression level, the CD9 expression level and/or the CD81 expression level of UMSC-EXO and UMSC-EXO-PD-L1-HGF were analyzed by the Western blotting, the ELISA and the flow cytometry. The primary antibodies used were PD-L1 (OriGene), HGF (Millipore), CD63 (Invitrogen), CD9 (ABclonal) and CD81 (ABclonal).

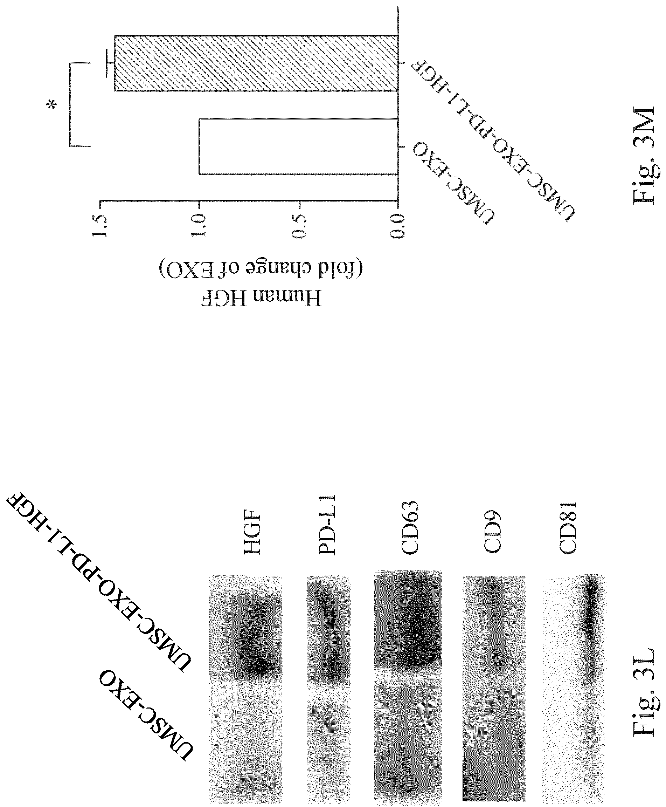

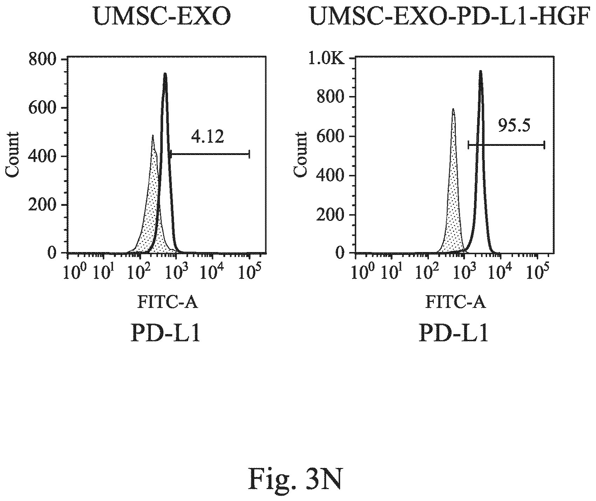

Reference is made to Fig. 3L to Fig. 3N, which show analysis results of the HGF content, the PD-L1 expression level, the CD63 expression level, the CD9 expression level and/or the CD81 expression level of UMSC-EXO and UMSC-EXO-PD-L1-HGF. Fig. 3L shows analysis results of the Western blotting, Fig. 3M shows analysis results of the ELISA of the HGF content, and Fig. 3N shows analysis results of the flow cytometry of the PD-L1 expression level. The results in Fig. 3L show that both UMSC-EXO and UMSC-EXO-PD-L1-HGF express specific exosomal markers CD9, CD63 and CD81, confirming the identity of the exosome. Moreover, the PD-L1 expression level and the HGF content in UMSC-EXO-PD-L1-HGF are more than that in UMSC-EXO. The results in Fig. 3M show that the HGF content in UMSC-EXO-PD-L1-HGF is significantly higher than that in UMSC-EXO, which are consistent with the results in Fig. 3L. The results in Fig. 3N show that the PD-L1 expression level in UMSC-EXO-PD-L1-HGF is significantly higher than that in UMSC-EXO, which are also consistent with the results in Fig. 3L.

Using the flow cytometry, the multiple production batches of EXO-PD-L1-HGF or UMSC-EXO-PD-L1-HGF can be analyzed and quantified, and the exosome with PD-L1 surface markers can be measured and quantified to define the proportion of the PD-L1 surface markers that the therapeutic exosome should possess.

Using the ELISA, the multiple production batches of EXO-PD-L1-HGF or UMSC-EXO-PD-L1-HGF can be analyzed and quantified, and the HGF content can be measured and quantified to define the HGF content that the therapeutic exosome should possess.

### II. Regulatory mechanism of the exosome of the present disclosure

### 2.1 The exosome of the present disclosure significantly attenuates the H₂O₂-induced neuronal apoptosis in vitro

In order to study whether EXO-PD-L1-HGF attenuates H₂O₂-induced cell death, the cells used in this test experiment were primary cortical cells (PCCs) and human SH-SY5Y cells (hereinafter referred to as SH-SY5Y). The PCCs were prepared from embryonic-day18 (E18) C57BL/6JNarl mice. Briefly, isolated cerebral cortex were pooled, dissociated via mechanical trituration, and suspended in growth medium (consisting of Neurobasal medium, 2% B-27 supplement, 0.5 mM L-glutamine, and 25 µM glutamic acid). The cells were counted using trypan blue exclusion, and then plated on either poly-d-lysine-coated 24-well or 6-well plates (Nunc) at a density of 3×10⁴ cells/24-well (cortex) or 2-3×10⁶ cells/6-well. The cultures were maintained in a humidified incubator with 5% CO₂ at 37°C, and after 3 days *in vitro* (DIV 3) half of the growth medium was removed and replaced with maintenance medium (consisting of Neurobasal medium, 2% B-27 supplement and 0.5 mM L-glutamine). Maintenance medium was changed in the same method every 3-4 days. The current method produced cultures wherein > 95% of the cells was neurons. The SH-SY5Y were cultured in DMEM/F12 (1:1, v:v) medium (Gibco) with 10% fetal bovine serum (FBS) and 1% penicillin-streptomycin, and were grown in a humidified atmosphere of 5% CO₂ at 37°C.

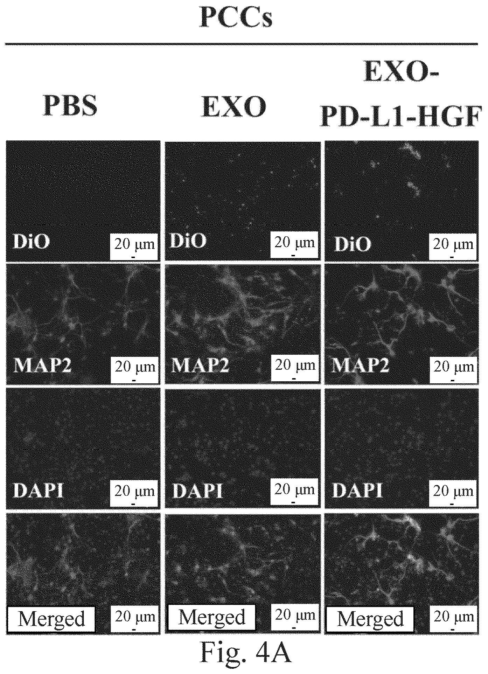

First an immunofluorescence staining was used to examine cellular uptake of the exosome by the PCCs *in vitro.* EXO and EXO-PD-L1-HGF were labeled with 1 mg/mL DiO green dye (Invitrogen) for 1 hour at RT and re-pelleted by using Total Exosome Isolation reagent for overnight at 4°C. And centrifuged at 10,000×g for 60 minutes at 4°C, further fluorescently labeled exosomes (hereinafter referred to as DiO-EXO and DiO-EXO-PD-L1-HGF) were resuspended by sterile 1×PBS. Cellular nuclei were stained using 4,6 diamidino-2-phenylindole (DAPI). After incubation of DiO-EXO and DiO-EXO-PD-L1-HGF with DiD (Invitrogen)-labeled cells for 1 hour at 37°C, images of the exosome uptake were acquired with a Confocal Spectral Microscope Leica SP2. Reference is made to Fig. 4A, which shows analysis results of DiO-EXO and DiO-EXO-PD-L1-HGF uptake by the PCCs. The results in Fig. 4A show that white tiny spots of DiO-EXO-PD-L1-HGF and DiO-EXO were endocytosed into the cytoplasm of MAP2⁺ PCCs.

When measuring H₂O₂-induced cell death, the PCCs or the SH-SY5Y were seeded at a cell density of 3x10⁵ cells/24-well plate or 1x10⁵ cells /24-well plate for overnight, and at the 24th hour treated with various concentrations of H₂O₂ (0 µM, 100 µM, 200 µM, 300 µM, 400 µM and 500 µM) for 3 hours in the 37°C with 5% CO₂, respectively. Then the cell viability was measured by a CCK-8 assay to determine the optimal concentration and duration of H₂O₂ treatment to induce cytotoxicity on the PCCs and the SH-SY5Y.

Based on the results, H₂O₂ treatment significantly induced cell death of the PCCs and the SH-SY5Y in a concentration-dependent manner, and the optimal treatment dose was 300 µM (data not shown). Therefore, the PCCs or the SH-SY5Y were pre-treated with 20 µg of EXO or EXO-PD-L1-HGF for 24 hours, and then treated with 300 µM H₂O₂ for 3 hours. The CCK-8 assay was used to measure cell viability to study whether the exosome of the present disclosure can exert a neuroprotective effect under H₂O₂-induced cell death, and a TUNEL staining was used to detect cell apoptosis.

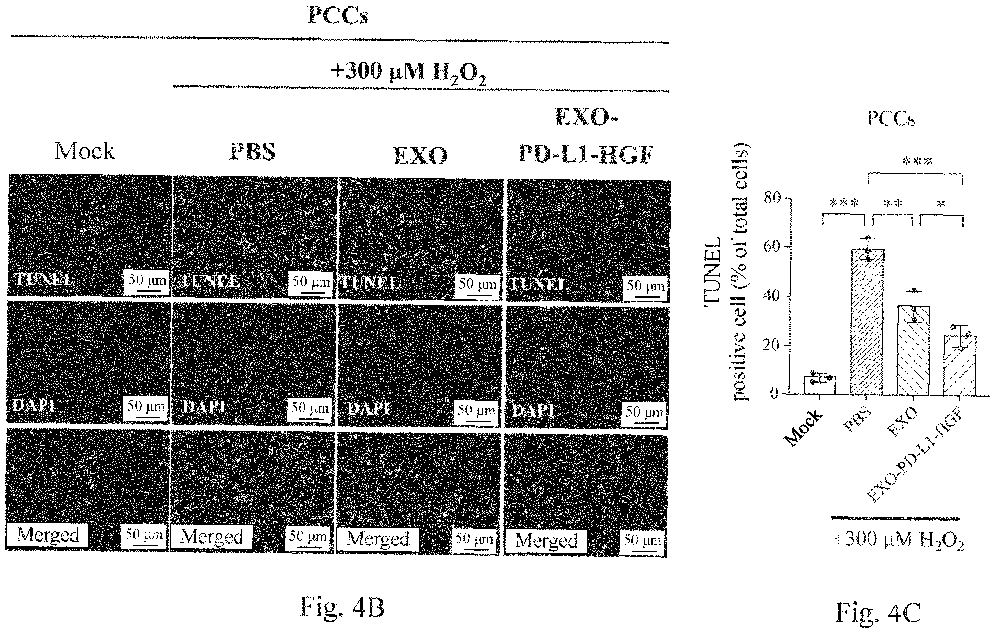

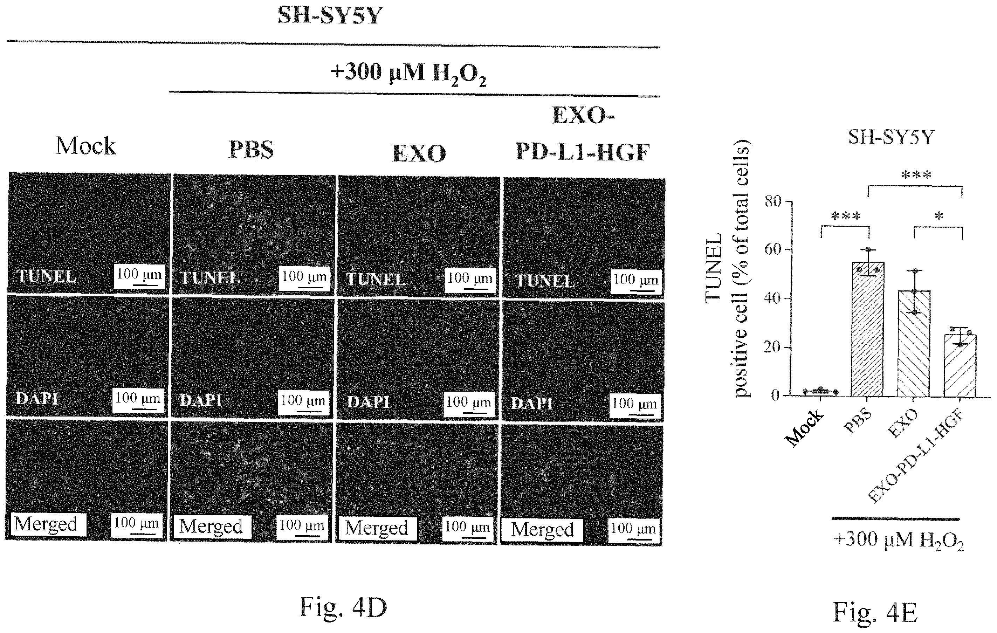

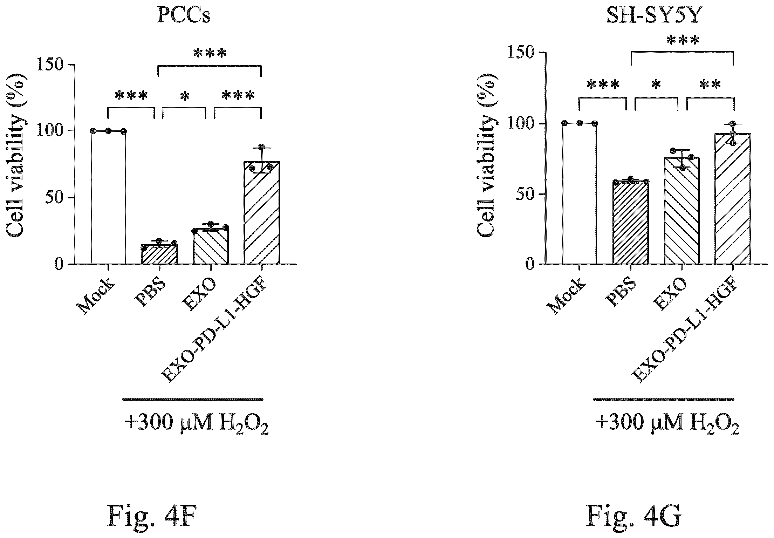

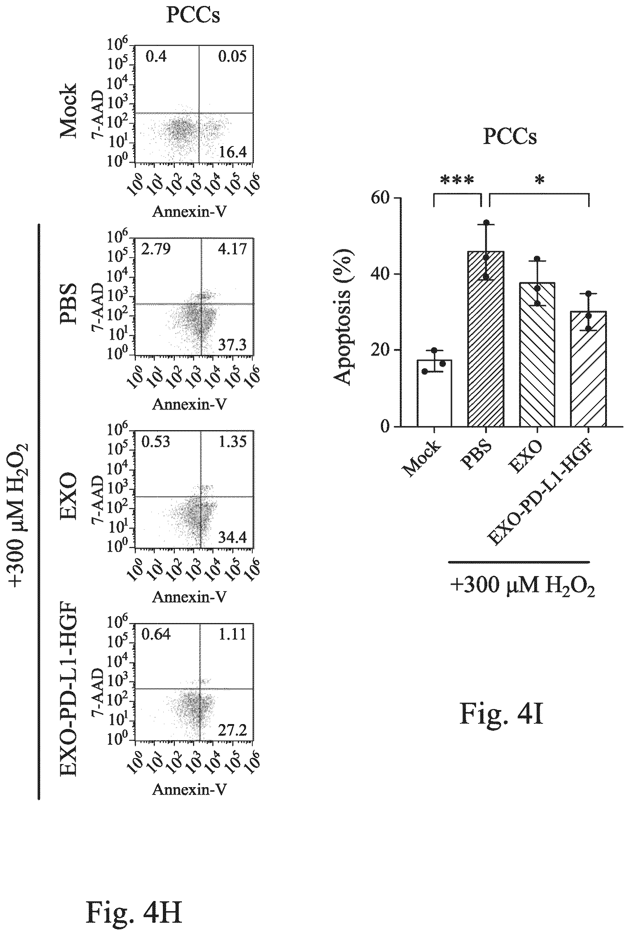

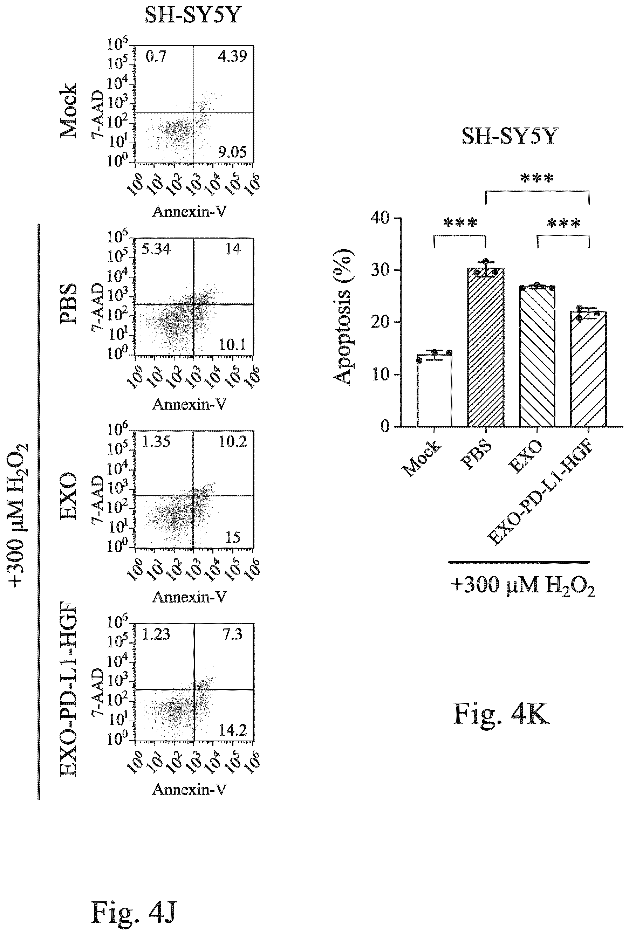

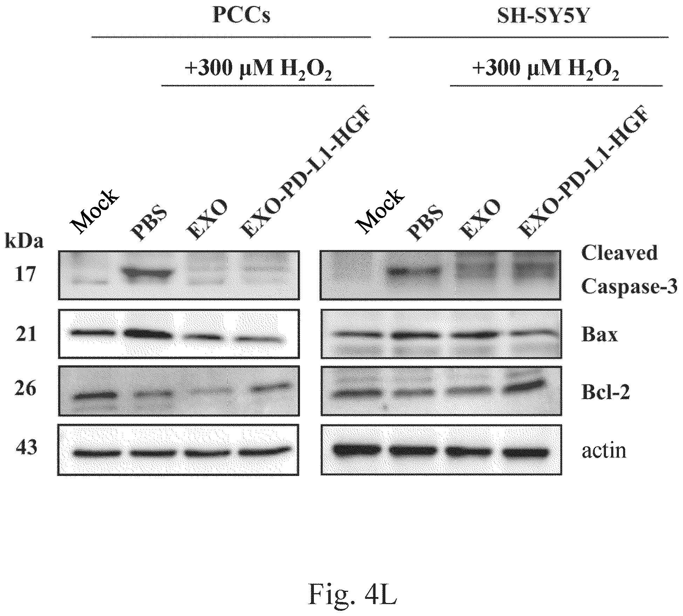

Reference is made to Fig. 4B to Fig. 4L. Fig. 4B and Fig. 4C show analysis results of the TUNEL staining to detect that EXO-PD-L1-HGF reduces H₂O₂-induced apoptosis in the PCCs *in vitro,* wherein Fig. 4C shows the statistical results of Fig. 4B. Fig. 4D and Fig. 4E show analysis results of the TUNEL staining to detect that EXO-PD-L1-HGF reduces H₂O₂-induced apoptosis in the SH-SY5Y *in vitro,* wherein Fig. 4E shows the statistical results of Fig. 4D. Fig. 4F and Fig. 4G respectively show analysis results of the CCK-8 assay to detect that EXO-PD-L1-HGF reduces H₂O₂-induced cell death of the PCCs and the SH-SY5Y *in vitro.* Fig. 4H and Fig. 4I show analysis results of an Annexin V-FITC/7-AAD double staining to detect that EXO-PD-L1-HGF reduces H₂O₂-induced apoptosis in the PCCs *in vitro,* wherein Fig. 4I shows the statistical results of Fig. 4H. Fig. 4J and Fig. 4K show analysis results of the Annexin V-FITC/7-AAD double staining to detect that EXO-PD-L1-HGF reduces H₂O₂-induced apoptosis in the SH-SY5Y *in vitro,* wherein Fig. 4K shows the statistical results of Fig. 4J. Fig. 4L shows analysis results of the Western blotting of the expression of apoptosis-related proteins in H₂O₂-induced apoptosis of the PCCs and the SH-SY5Y after the pretreatment with EXO or EXO-PD-L1-HGF. In Fig. 4B to Fig. 4L, "Mock" represents the untreated group, "PBS" represents the group pretreated with PBS and then treated with H₂O₂, "EXO" represents the group pretreated with EXO and then treated with H₂O₂, and "EXO-PD-L1-HGF" represents the group pretreated with EXO-PD-L1-HGF and then treated with H₂O₂. Results are presented as means ± SD, wherein * represents p<0.05, ** represents p<0.01, and *** represents p<0.001.

The results in Fig. 4B to Fig. 4E show that pretreatment with EXO-PD-L1-HGF for overnight significantly reduced the H₂O₂-induced apoptotic ratio (TUNEL positive cells showed white dots) compared with the EXO group and the PBS group in both the PCCs and the SH-SY5Y. The CCK-8 assay results in Fig. 4F and Fig. 4G show that more reduction of H₂O₂-induced cell death was demonstrated in pretreatment with 20 µg of EXO-PD-L1-HG than in the EXO group and the PBS group, wherein the cell survival rate of the PCCs could be increased to 80% and the cell survival rate of the SH-SY5Y could be increased to 85%. The results in Fig. 4H to Fig. 4K show that pretreatment with EXO-PD-L1-HGF significantly mitigated apoptosis after exposure to H₂O₂ compared with the EXO group and the PBS group. Furthermore, the results in Fig. 4L show that compared with the EXO group and the PBS group, expression levels of the pro-apoptotic proteins Bax and Cleaved Caspase-3 were significantly decreased whereas anti-apoptotic protein Bcl-2 expression was elevated on the PCCs and the SH-SY5Y in pretreatment with EXO-PD-L1-HGF for 24 hours prior to H₂O₂ addition, which was similar to that of the Mock group.

Whether UMSC-EXO-PD-L1-HGF could attenuate H₂O₂-induced cell death was also measured. The PCCs were seeded at a cell density of 3x10⁵ cells/24-well plate for overnight, and at the 24th hour treated with various concentrations of H₂O₂ (0 µM, 100 µM, 200 µM, 300 µM, 400 µM and 500 µM) for 3 hours in the 37°C with 5% CO₂, respectively. Then the cell viability was measured by the CCK-8 assay. Based on the results, the optimal treatment dose was 200 µM (data not shown), and the treatment dosage of 200 µM H₂O₂ was selected in subsequent experiments. In order to study whether the exosome of the present disclosure can exert the neuroprotective effect under H₂O₂-induced cell death, the PCCs were pretreated with 20 µg of UMSC-EXO or UMSC-EXO-PD-L1-HGF for 24 hours, and then treated with 200 µM H₂O₂ for 3 hours. The CCK-8 assay was used to measure cell viability, and the TUNEL staining was used to detect cell apoptosis.

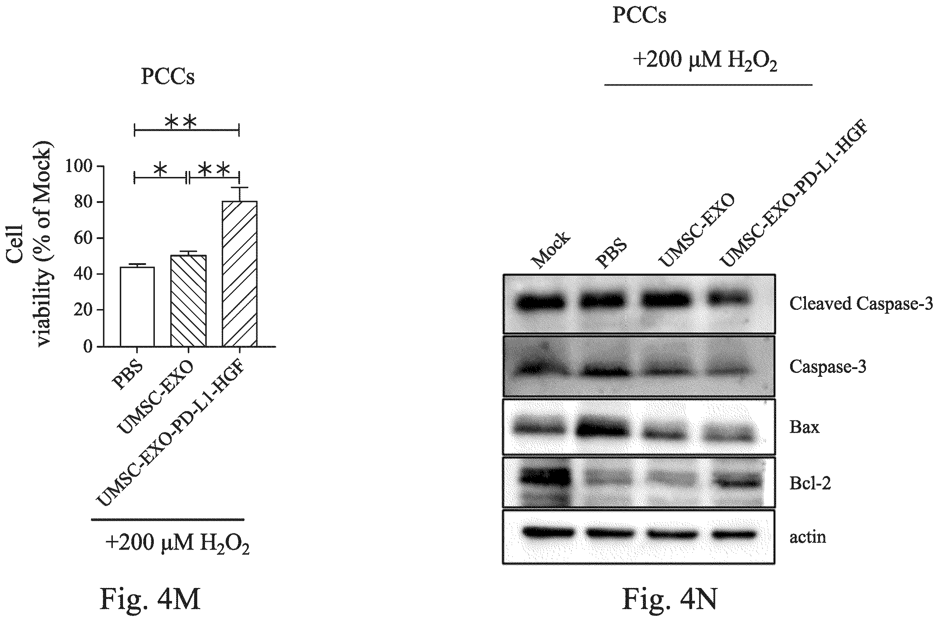

Reference is made to Fig. 4M and Fig. 4N. Fig. 4M shows analysis results of the CCK-8 assay to detect that UMSC-EXO-PD-L1-HGF reduces H₂O₂-induced cell death *in vitro,* and results are presented as means ± SD, wherein * represents p<0.05, and ** represents p<0.01. Fig. 4N shows analysis results of the Western blotting of the expression of apoptosis-related proteins in H₂O₂-induced apoptosis of the PCCs cells after the pretreatment with UMSC-EXO-PD-L1-HGF.

The results in Fig. 4M show that more reduction of H₂O₂-induced cell death was demonstrated in pretreatment with 20 µg of UMSC-EXO-PD-L1-HG than in the UMSC-EXO group and the PBS group, wherein the cell survival rate of the PCCs could be increased to 80%. The results in Fig. 4N show that compared with the UMSC-EXO group and the PBS group, expression levels of the pro-apoptotic proteins Bax and Cleaved Caspase-3 were significantly decreased whereas anti-apoptotic protein Bcl-2 expression was significantly increased. Collectively, the above results suggest that the exosome of the present disclosure owned neuroprotective ability against H₂O₂ induced cytotoxicity.

### 2.2 The exosome of the present disclosure suppresses cytotoxic T cells and promotes Treg cells activation

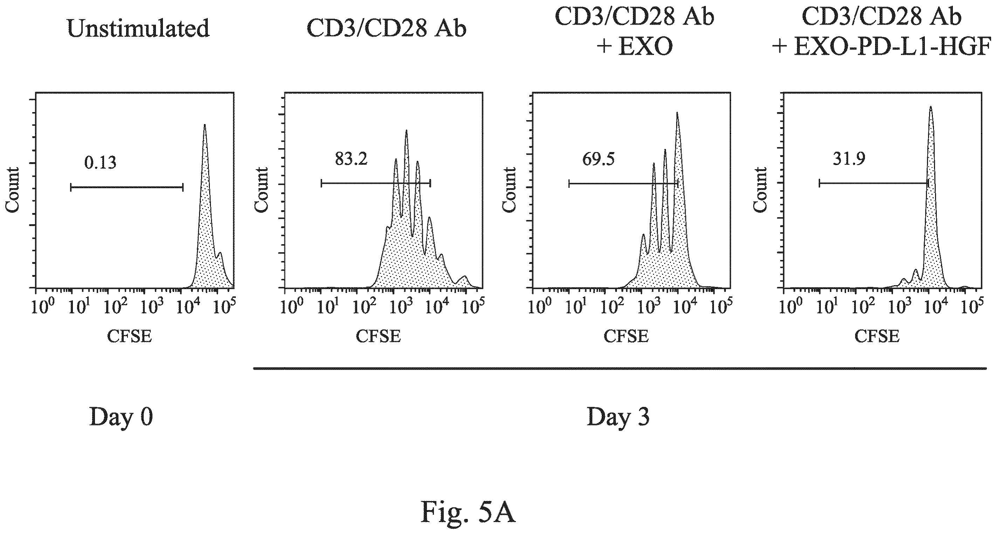

To investigate whether EXO-PD-L1-HGF affects the proliferation of T cells *in vitro,* CD3/CD28 antibody-stimulated proliferating T cells were stained with CFSE Cell Proliferation Kit (Invitrogen), treated with EXO or EXO-PD-L1-HGF for 3 days, and then the fluorescent intensity of CFSE-labeled cells was measured by the flow cytometry. Reference is made to Fig. 5A, which shows results of the CFSE assay to analyze that EXO-PD-L1-HGF suppresses the proliferation of T cells, wherein "Unstimulated" represents the untreated group, "CD3/CD28 Ab" represents the group stimulated only with CD3/CD28 antibodies (hereinafter referred to as "vehicle control group"), "CD3/CD28 Ab + EXO" represents the group stimulated with CD3/CD28 antibodies and then treated with EXO (hereinafter referred to as "EXO group"), "CD3/CD28 Ab + EXO-PD-L1-HGF" represents the group treated with CD3/CD28 antibodies and then treated with EXO-PD-L1-HGF (hereinafter referred to as "EXO-PD-L1-HGF group"). The results in Fig. 5A show that the percentage of T cell proliferation significantly diminished in the EXO-PD-L1-HGF group compared with the other groups.

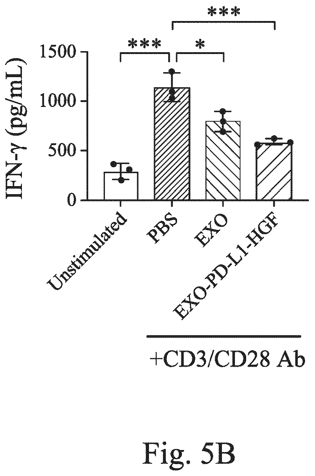

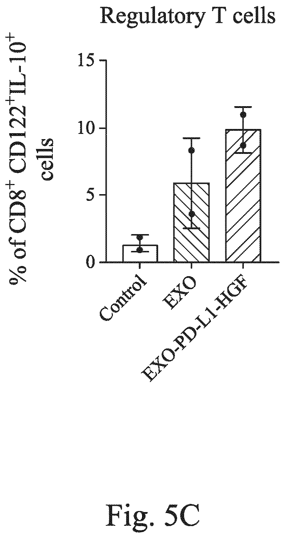

To assess the activation of T cells, the interferon-γ (IFN-γ) expression in all groups was measured by the ELISA. Reference is made to Fig. 5B, which shows results of the ELISA to analyze the reduction of the IFN-γ expression by EXO-PD-L1-HGF. The results in Fig. 5B show that the IFN-γ expression of T cells in the EXO-PD-L1-HGF group was significantly reduced. Moreover, the percentage of regulatory T cells (CD8⁺CD122⁺IL-10⁺) in T cells treated with EXO or EXO-PD-L1-HGF was measured by the flow cytometry. Reference is made to Fig. 5C, which shows analysis results of the percentage of regulatory T cells after the treatment with EXO or EXO-PD-L1-HGF. The results showed that the number of regulatory T cells was upregulated in the EXO-PD-L1-HGF group. Taken together, these results validate that the exosome of the present disclosure was capable of exert immunosuppressive effect.

### 2.3 The exosome of the present disclosure promotes the proliferation of primary neural progenitor cells (NPCs) in vitro

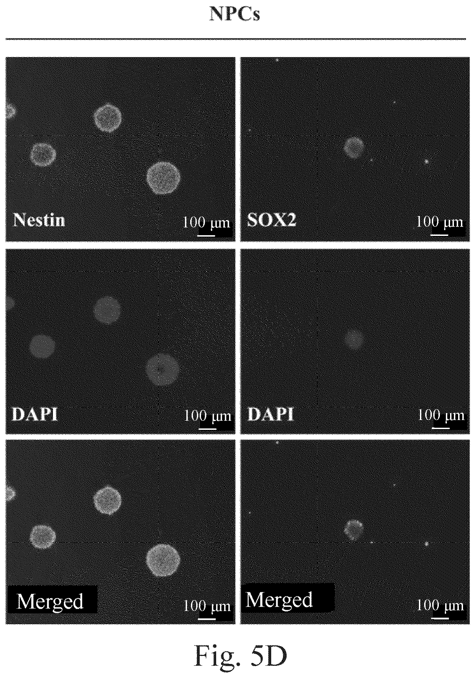

In order to verify the potential of EXO-PD-L1-HGF on the proliferation of the NPCs, the NPCs were first isolated from cerebral cortex of embryonic-day 17 (E17) C57BL/6JNarl mice. The isolation method was aseptically isolated and dissociated the collected cerebral cortex. Then, neurosphere cultures were prepared in Neurobasal medium (Gibco BRL) supplemented with 2% B-27 (Gibco BRL), 0.5 mM L-glutamine (PAN-Biotech), and 100 U/mL penicillin/0.1 mg/L streptomycin (Gibco BRL). For maintenance and expansion of the neurosphere cultures, Neurobasal medium was further supplemented with 20 ng/mL bFGF (Gibco BRL) and 20 ng/mL epidermal growth factor (EGF) (Gibco BRL). The neurosphere cultures were maintained at 37°C in a humidified incubator with 5% CO₂. To create clonal and low-density cultures, primary neurospheres grown at a high density were dissociated into single cell suspension and reseeded into 6-well plates at 1×10⁵ cells per mL of medium, and the newly formed secondary neurospheres were counted after 7 days. The stem cell markers such as Nestin expression and Sox2 expression of isolated NPCs were analyzed by the immunofluorescence staining. Reference is made to Fig. 5D, which shows analysis results of stem cell marker expression of isolated NPCs. The results in Fig. 5D showed that Nestin protein and Sox2 protein were expressed in the NPCs isolated by the above method.

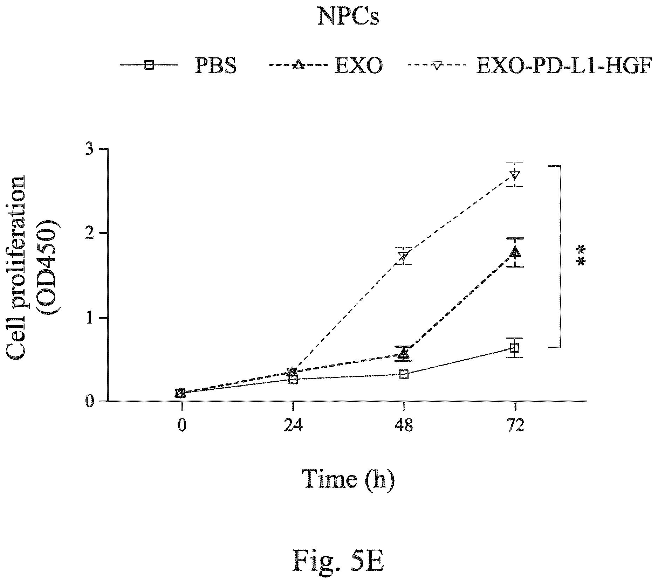

Next, the NPCs were treated with PBS, EXO and EXO-PD-L1-HGF for 72 hours, and then the cell viability was measured by the CCK-8 assay. Reference is made to Fig. 5E, which shows analysis results of the CCK-8 assay to detect that EXO-PD-L1-HGF promotes the proliferation of the NPCs. The results in Fig. 5E show revealed higher proliferation of the NPCs in the EXO-PD-L1-HGF group than that of the PBS group and the EXO group. Collectively, these results indicate that the exosome of the present disclosure significantly promoted the proliferation ability of the NPCs.

### 2.4 The exosome of the present disclosure positively regulates STAT3/FOXO3 signaling pathway involved in NPCs-induced neuroregeneration

In this test example, the potential mechanism of the exosome of the present disclosure participating in the neurotrophic effect of the NPCs was further verified. The NPCs were treated with EXO and EXO-PD-L1-HGF for 24 hours, and total RNA extraction was performed using Quick-RNA miniPrep kit (Zymo). Then RNA-based next-generation sequencing (NGS) (hereinafter referred to as RNA-seq) was used to analyze the total mRNA transcript expression of the NPCs in the control group, the EXO group (treated with EXO) and the EXO-PD-L1-HGF group (treated with EXO-PD-L1-HGF). RNA-seq results were delivered as BAM files which were converted to FASTQ format using Picard. FASTQ files were aligned to the hg19 human reference genome using Top hat 2.0.14. Expression values were calculated with feature Counts v1.4.6-p2, and differential expression analysis was determined by DESeq2. The downregulated genes for RNA-seq were then defined by counts > 100, folds > 2 and p < 0.05. Go analysis was performed using Gene Ontology Consortium website.

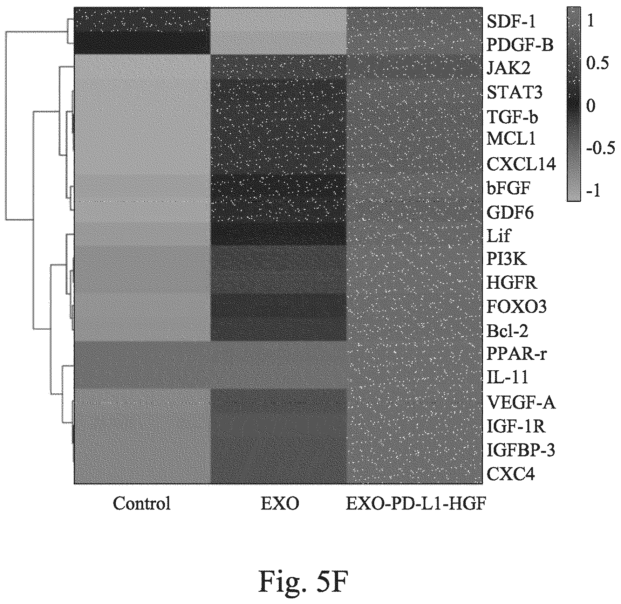

Reference is made to Fig. 5F, which is a heatmap of RNA-seq analysis of the control group, the EXO group, and the EXO-PD-L1-HGF group. The results in Fig. 5F show that the EXO-PD-L1-HGF group significantly upregulated several growth factors, chemokines, apoptosis genes and transcription factors, including hepatocyte growth factor receptor (HGFR), vascular-endothelial growth factor (VEGF), basic fibroblast growth factor (bFGF), stromal cell-derived factor-1 (SDF-1), CXCR4, signal transducer and activator of transcription 3 (STAT3) and FOXO3, etc. (fold change [FC] ≥2). Using a fold change [FC] ≥2 and a p-value <0.05 as a rule to determine differently expressed genes between the EXO-PD-L1-HGF group and the control group, there were about 613 upregulated genes in this pool (data not shown).

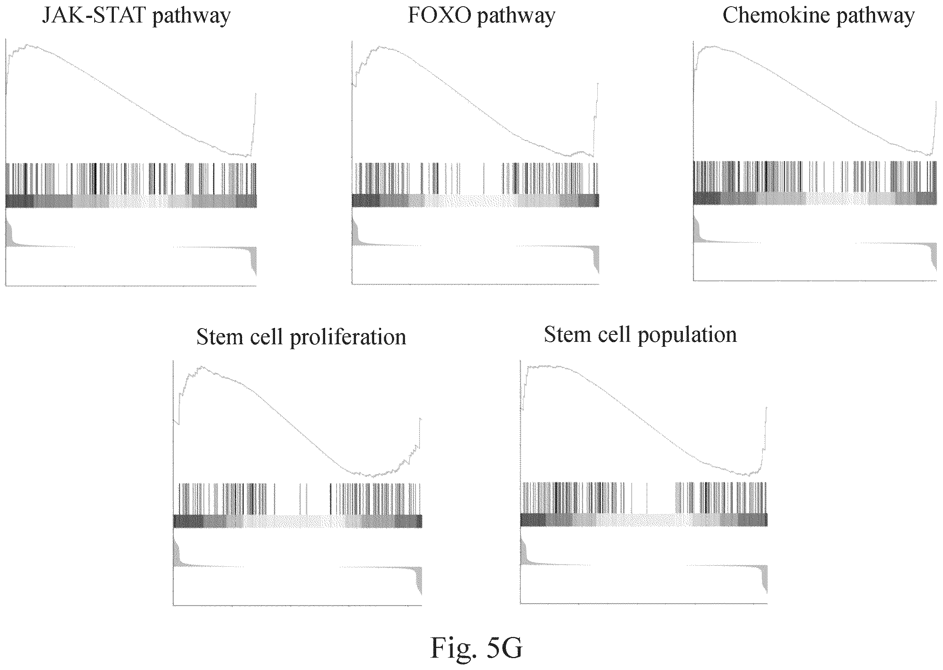

Moreover, a Gene Set Enrichment Analysis (GSEA) was performed to identify biological pathways that were significantly altered after EXO-PD-L1-HGF treatment (p-value <0.05). Reference is made to Fig. 5G, which shows results of GSEA of the control group, the EXO group and the EXO-PD-L1-HGF group. The results in Fig. 5G show that several biological pathways related to JAK-STAT signaling pathway, FOXO signaling pathway, chemokine signaling pathway, stem cell proliferation, and stem cell population maintenance were found to be significantly up-regulated in the EXO-PD-L1-HGF group.

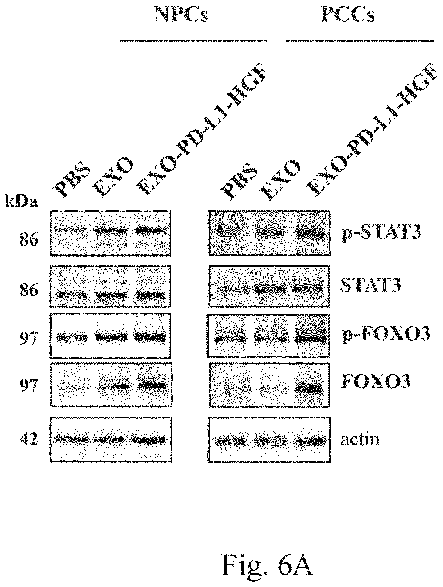

Furthermore, the NPCs and the PCCs were treated with PBS, EXO and EXO-PD-L1-HGF for 5 hours respectively. Cell lysate and total protein were collected using 1×RIPA lysis buffer (Sigma-Aldrich), and the protein expressions of phosphorylated STAT3 (hereinafter referred to as p-STAT3) and phosphorylated FOXO3 (hereinafter referred to as p-FOXO3) in the NPCs and the PCCs of the PBS group, the EXO group and the EXO-PD-L1-HGF group were detected by the Western blotting. The primary antibodies used were p-STAT3 (cell signaling), STAT3 (cell signaling), p-FOXO3 (cell signaling), FOXO3 (cell signaling) and actin (Millipore), where actin was the internal control. Reference is made to Fig. 6A, which shows results of the Western blotting of the expression of p-STAT3 and p-FOXO3 in the PCCs and the NPCs of the PBS group, the EXO group and the EXO-PD-L1-HGF group. The results show that treatment of EXO-PD-L1-HGF could significantly increase the protein expressions of p-STAT3 and p-FOXO3 in cells, whether in the PCCs or the NPCs.

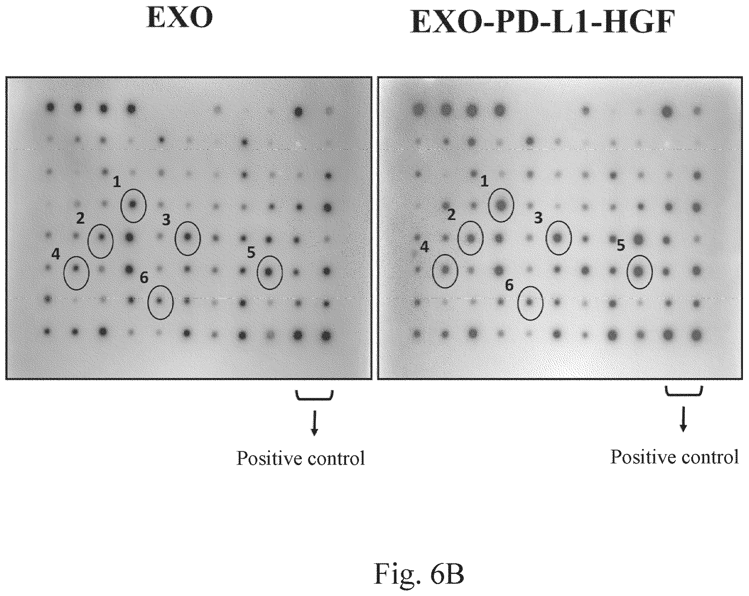

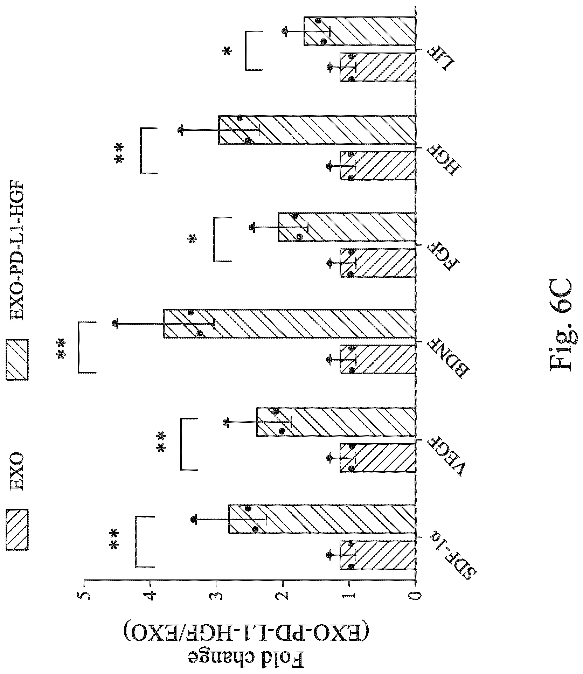

To systemically investigate the modulating molecules encapsulated in the exosome of the present disclosure, human cytokine arrays were performed to analyze the important agents containing in EXO and EXO-PD-L1-HGF. Since mesenchymal stem cells can exert immunomodulatory effect through paracrine secretion (e.g., IL-10 and TGFβ), the conditioned medium of the PCCs treated with EXO or EXO-PD-L1-HGF was collected. Exosomal proteins were extracted using lysis buffer supplemented with a protease and phosphatase inhibitor cocktail, and then analyzed using a human cytokine array (R&D Systems). Reference is made to Fig. 6B, which shows results of the human cytokine array to analyze the protein expression of cytokines in the PCCs after the treatment with EXO or EXO-PD-L1-HGF. The results show that the expression levels of 6 cytokines in the EXO-PD-L1-HGF group were significantly higher than those in the EXO group, including SDF-1α (circle 1), VEGF (circle 2), brain-derived neurotrophic factor (BDNF; circle 3), fibroblast growth factor (FGF; circle 4), HGF (circle 5), and leukemia inhibitory factor (LIF; circle 6). Furthermore, the expression levels of cytokines in the EXO group and the EXO-PD-L1-HGF group were detected by the ELISA. Reference is made to Fig. 6C, which shows results of the ELISA to analyze the protein expression of cytokines in the PCCs after the treatment with EXO or EXO-PD-L1-HGF. The results in Fig. 6C confirm that the expression levels of SDF-1α, VEGF, BDNF, FGF, HGF and LIF in the EXO-PD-L1-HGF group were indeed significantly higher than those in the EXO group. Taken together, the results suggest that the exosome of the present disclosure has the potential to suppress the detrimental injury to rescue neural damage.

In order to further confirm whether FOXO3 plays an important role in neuroprotection and neuroregeneration, FOXO3 shRNA (purchased from RNAi Core Center, hereafter referred to as sh-FOXO3) and scrambled GFP shRNA (purchased from RNAi Core Center, hereafter referred to as sh-control) was cloned into the expression vector pLKO1. The plasmid and lentiviral packaging constructs, pCMV-ΔR8.91 and pMD.G were co-transfected in HEK293T cells via lipofectamine 3000 transfection reagent (Invitrogen). After 24-48 hours, lentiviral particles of sh-FOXO3 (LV-sh-FOXO3) and lentiviral particles of sh-control (LV-sh-control) were collected through 0.45 mm filters and concentrated by spinning at 4,000×g for 15 minutes followed by a second spin at 1,000×g for 2 minutes at room temperature for further experiment. The concentrated viruses were used freshly or stored at -80°C.

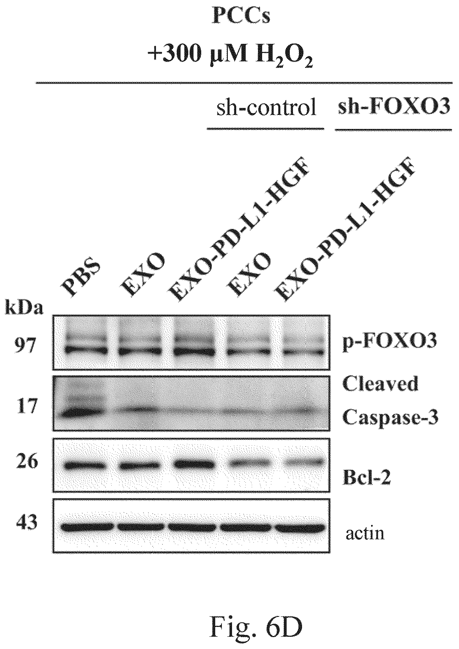

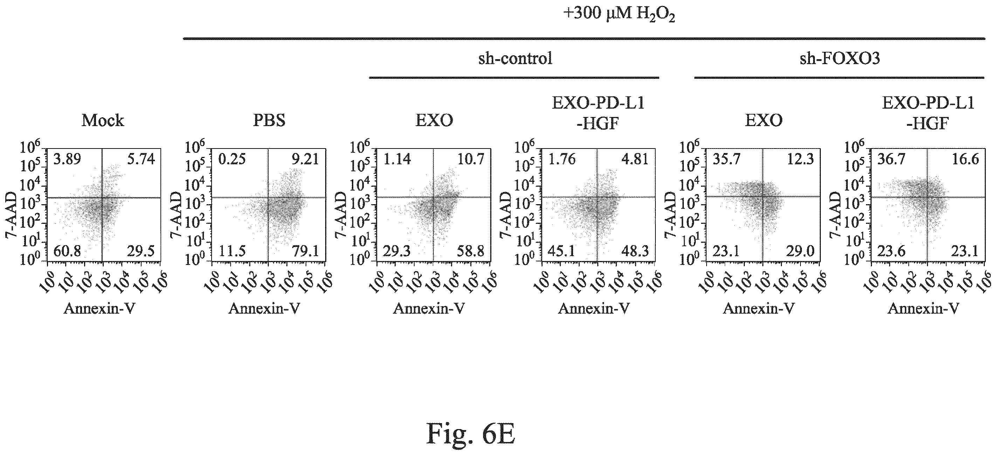

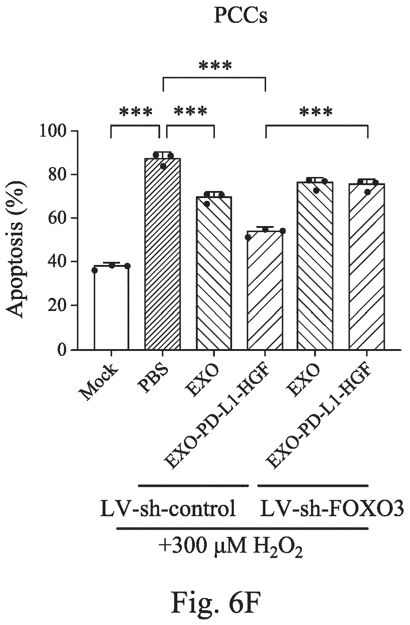

The PCCs were infected with LV-sh-FOXO3 or LV-sh-control respectively, pretreated with EXO and EXO-PD-L1-HGF respectively, and then treated with 300 µM H₂O₂. The expression levels of apoptosis-related proteins in the PCCs in all groups were analyzed by the Western blotting and the Annexin V-FITC/7-AAD double staining. Reference is made to Fig. 6D, which shows analysis results of the Western blotting of the expression of apoptosis-related proteins in the PCCs with the knockdown FOXO3 expression after the treatment with the exosome of the present disclosure. The results show that knockdown FOXO3 level reduced the increased protein expression of p-FOXO3 and Bcl-2 after treatment with EXO-PD-L1-HGF. Reference is made to Fig. 6E and Fig. 6F, which show results of the flow cytometry to analyze apoptosis in different groups. Fig. 6F shows the statistical results of Fig. 6E, and results are presented as means ± SD, wherein *** represents p<0.001. The results in Fig. 6E and Fig. 6F show that knockdown FOXO3 level significantly reduced the effect of EXO-PD-L1-HGF-attenuated neuronal apoptosis in the H₂O₂-induced PCCs.

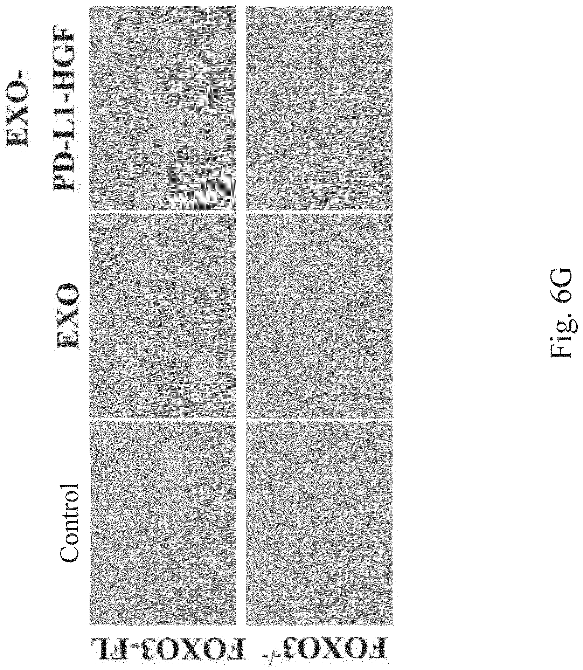

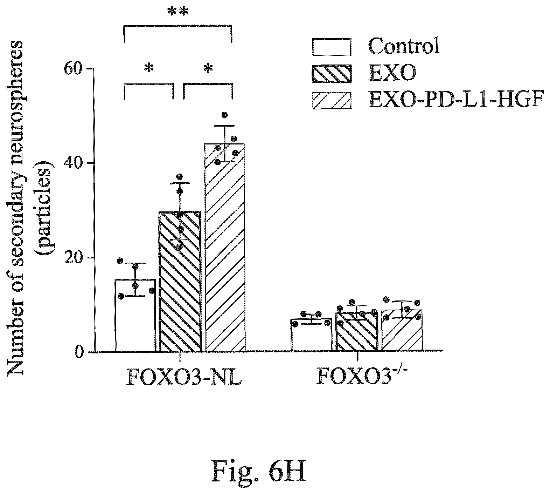

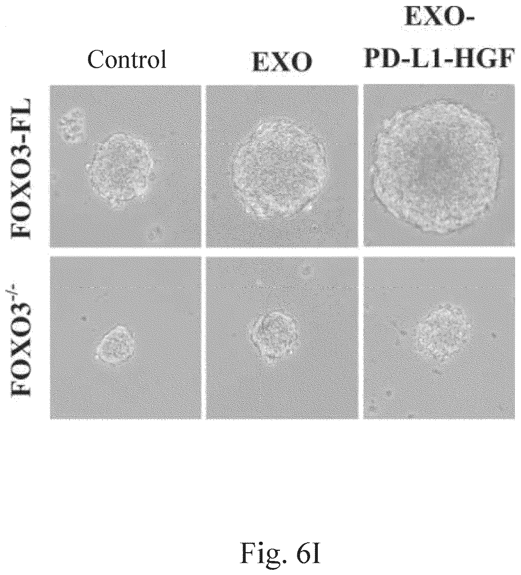

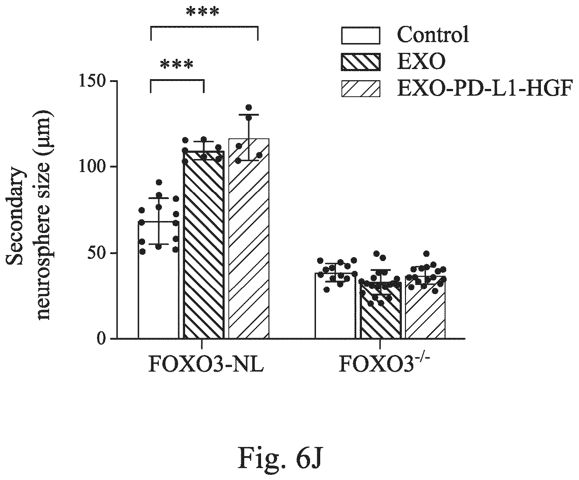

To verify the potential of EXO-PD-L1-HGF on the proliferation of the NPCs through the *FOXO3* gene, the NPCs were isolated from cerebral cortex of embryonic-day 17 (E17) of FOXO3-NL (genotype FOXO3^{+/+}, hereafter referred to as FOXO3-NL) mice and *FOXO3* gene knockout mice (genotype FOXO3^{-/-}, hereafter referred to as FOXO3^{-/-}). The self-renewal ability of the isolated NPCs was further analyzed, including analyzing the number of secondary neurospheres formed by each of the NPCs and measuring the size of the secondary neurospheres formed. Reference is made to Fig. 6G to Fig. 6J. Fig. 6G and Fig. 6H show analysis results of the number of secondary neurospheres formed by different groups of the NPCs, and Fig. 6H shows the statistical results of Fig. 6G. Fig. 6I and Fig. 6J show analysis results of the secondary neurospheres size formed by different groups of the NPCs, and Fig. 6J shows the statistical results of Fig. 6I. Results in Fig. 6H and Fig. 6J are presented as means ± SD, wherein * represents p<0.05, ** represents p<0.01, and *** represents p<0.001. The results show that knockout FOXO3 level significantly suppressed the EXO-PD-L1-HGF-promoted NPCs proliferation by the number of secondary NPCs and the secondary NPCs size.

### III. The exosome and pharmaceutical composition of the present disclosure are used to treat the ischemia condition of the tissue

The data of the aforementioned part test examples demonstrate that the exosome of the present disclosure has the ability to protect neurons against H₂O₂-induced cytotoxicity, can inhibit cytotoxic T cells and promote the activation of regulatory T cells, promote the proliferation of the NPCs, and positively regulate the STAT3/FOXO3 signaling pathway involved in neuroprotection and neuroregeneration. In this part test examples, ischemic stroke mice were used to evaluate and explore the effect of using the exosome of the present disclosure to treat the ischemia condition of the tissue, and detect the neurological behavior of mice before and after the stroke through two types of neurobehavioral assessments to assess whether neurological function had recovered.

An ischemia-reperfusion model was used to simulate symptoms of temporary localized cerebral ischemia in mice. Test animals were adult male C57BL/6 mice weighing 25-30 g, and were subjected to two-vessel ligation. All surgical procedures, animal experimental protocols, and methods were performed in accordance with Institutional Guidelines for Animal Research and approved by the Institutional Ethical Committee for Animal Research. Mice were anesthetized with chloral hydrate (0.4 g/kg, ip), and ligated the right middle cerebral artery (MCA) and common carotids arteries (CCAs). The CCAs were clamped with non-traumatic arterial clips. Using a surgical microscope, a 2×2 mm craniotomy was drilled where the zygoma fuses to the squamosal bone. The right MCA was ligated with a 10-0 nylon suture. Cortical blood flow (CBF) was measured continuously with a laser Doppler flowmeter (PF-5010, Periflux system) in anesthetized animals. A 1 mm burr hole was made in the right fronto-parietal region to place photodetectors. A probe (0.45 mm in diameter) was stereotaxically placed in the cortex (1.3 mm posterior, 2.8 mm lateral to the bregma, and 1.0 mm below the dura). After 120 minutes ischemia, the suture on the MCA and the arterial clips on CCAs were removed to allow reperfusion, to obtain middle cerebral artery occlusion (MCAO) mice (hereinafter referred to as MCAO mice). Experimental mice were then injected with EXO or EXO-PD-L1-HGF (200 µg in 100 µL PBS) or vehicle (100 µL PBS) 30 minutes after reperfusion through a 26-gauge syringe into the right femoral vein. During anesthesia, core body temperature was monitored with a thermistor probe and maintained at 37°C using a heating pad. After recovery, body temperature of each of the MCAO mice was maintained at 37°C with a heat lamp.

### 3.1 The exosome of the present disclosure are prominently migrated into ischemic brain

To examine the biodistribution and migration of EXO and EXO-PD-L1-HGF *in vivo,* lipophilic dye of DiD (Invitrogen) were performed to label on the surface of EXO and EXO-PD-L1-HGF. 50 µg exosome (EXO or EXO-PD-L1-HGF) were incubated with the DiD for 1 hour at 37°C. After staining, free dye was depleted by gel filtration using Exosome Spin Columns. The MCAO mice were randomly grouped and injected with fluorescently labeled exosome (DiD-EXO or DiD-EXO-PD-L1-HGF) via the femoral vain, wherein the injection dose per MCAO mouse is 1 mg/kg. Analysis was performed at 4 hours, 24 hours and 72 hours after injection. The MCAO mice were then anesthetized with 2% isoflurane in oxygen and the fluorescence images were acquired using an *in vivo* imaging system by IVIS of Living Image 3.0 software (Xenogen Corp.) (excitation: 745 nm and emission: 830 nm). After 72 hours, the MCAO mice were euthanized. The ROI tool was used to relatively quantify the fluorescence signal.

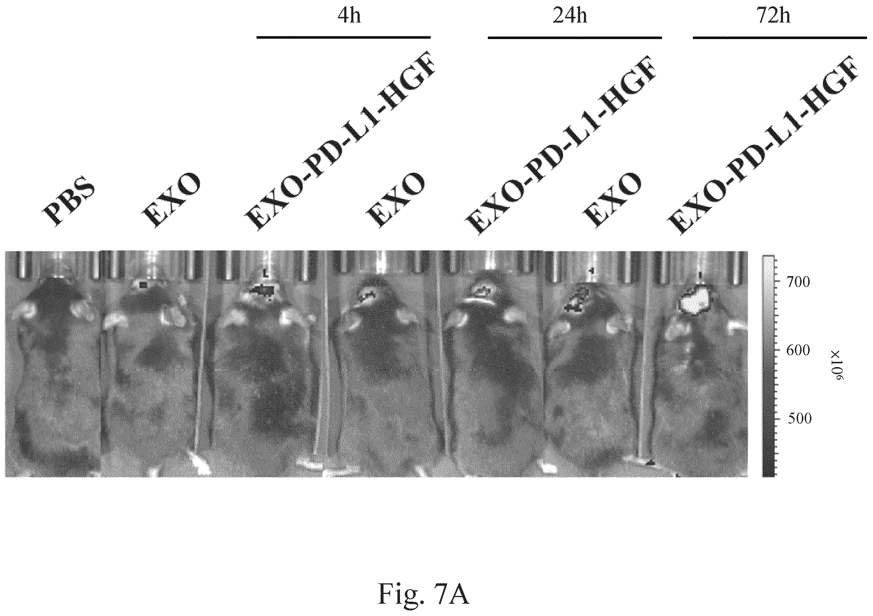

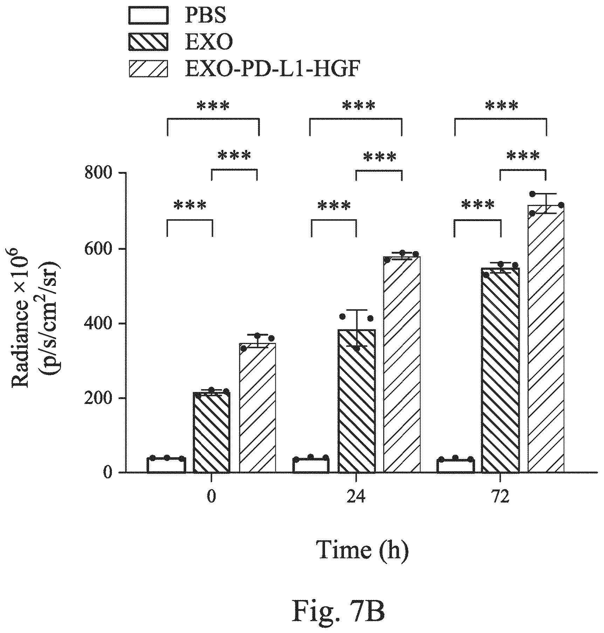

Reference is made to Fig. 7A and Fig. 7B, which show analysis results of fluorescence intensity after intravenous injection of DiD-EXO or DiD-EXO-PD-L1-HGF into the MCAO mice. Fig. 7B shows the statistical results of Fig. 7A, and results are presented as means ± SD, wherein *** represents p<0.001. The results in Figs. 7A and 7B show that the fluorescence signal of DiD-EXO and DiD-EXO-PD-L1-HGF in IVIS *in vivo* imaging system was detectable 4 hours after injection, followed by an obvious increase of the fluorescence intensity with detection time at the stroke site. Significant enhancement of fluorescent signal with a time-dependent manner was found in the stroke brain of the EXO-PD-L1-HGF group compared to the EXO group and the PBS group.

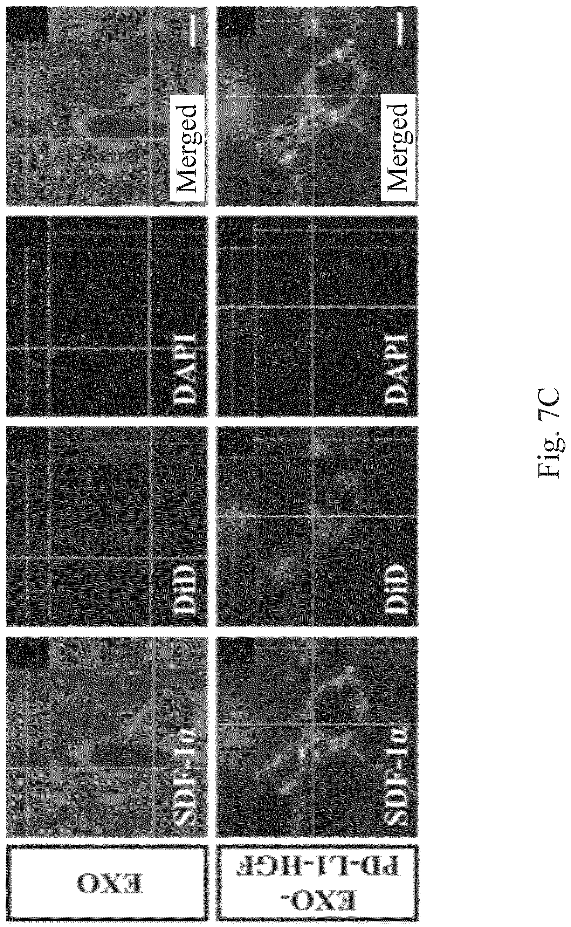

The brain tissues of the MCAO mice were also stained with an immunofluorescence staining to detect the ischemic damage area. Reference is made to Fig. 7C, which shows results of the immunofluorescence staining of EXO-PD-L1-HGF co-localized with SDF-1α at the injury site. The results in Fig. 7C show that more SDF-1α⁺ cells colocalized with DiD-EXO-PD-L1-HGF in the EXO-PD-L1-HGF group than that of the EXO group, which indicate the homing ability of EXO-PD-L1-HGF to the site of the ischemic injury may be related to SDF-1α expressed at the injury site of post-stroke.

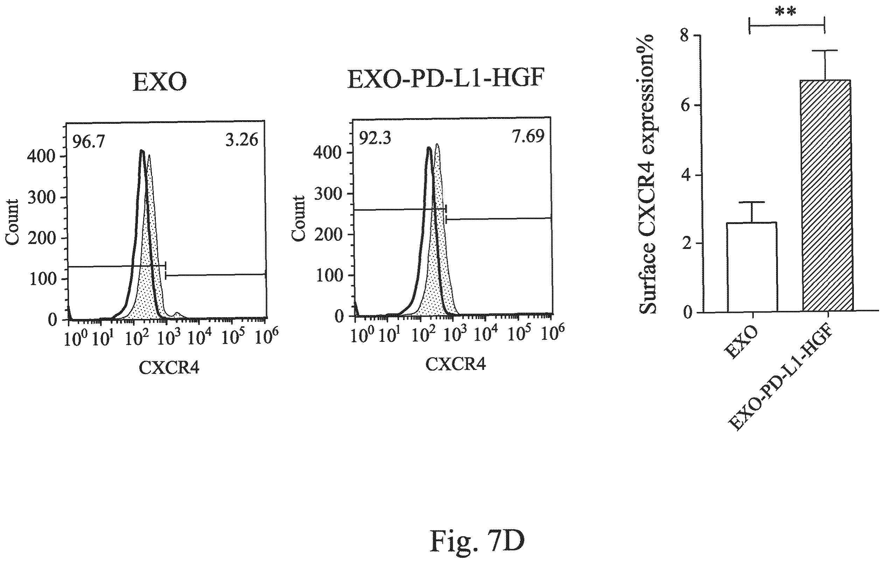

Previous studies showed SDF-1/CXCR4 plays a significant role in directing mesenchymal stem cell homing to the injury site. To investigate whether the homing effect of EXO-PD-L1-HGF is enhanced through increasing CXCR4 expression, the CXCR4 expression level on the exosome membrane of EXO and EXO-PD-L1-HGF were further analyzed by the flow cytometry. Reference is made to Fig. 7D, which shows analysis results of the flow cytometry to detect the CXCR4 expression level on exosome membrane of EXO and EXO-PD-L1-HGF. The results show that the CXCR4 expression level on the exosome membrane was significantly increased in EXO-PD-L1-HGF than that of EXO.

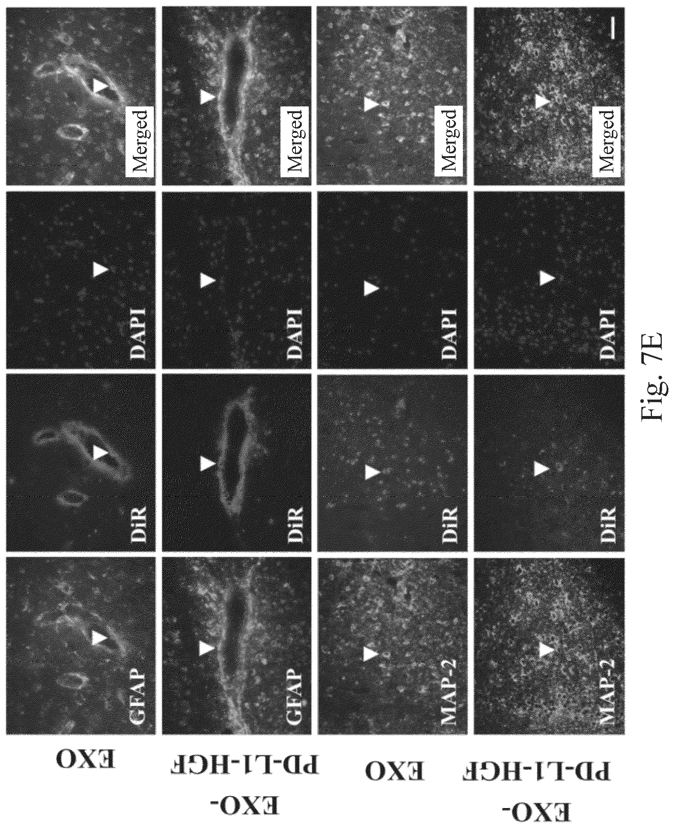

In addition, reference is made to Fig. 7E, which shows results of the immunofluorescence staining of EXO and EXO-PD-L1-HGF co-localized with GFAP⁺ cells and MAP2⁺ cells at the injury site. Fluorescent signal of DiD-EXO-PD-L1-HGF after intravenous injection into the MCAO mice at 72 hours post the ischemic stroke was observed to co-express with fluorescent signal of GFAP⁺ or MAP2⁺ cells in the stroke brain by the immunofluorescence staining. Taken together, these data indicate that implantation of the exosome of the present disclosure into the MCAO mice can home to the ischemic injury site, accumulate in the ischemic brain, and then be endocytosed into neuroglial cells to exert regenerative effect thereof.

Using the flow cytometry, the CXCR4 expression level on the exosome membrane of UMSC-EXO and UMSC-EXO-PD-L1-HGF can also be analyzed, and the differences in the exosome with CXCR4 surface markers between the UMSC-EXO-PD-L1-HGF group and the UMSC-EXO group can be measured and quantified.

Using the flow cytometry, the multiple production batches of EXO-PD-L1-HGF or UMSC-EXO-PD-L1-HGF can also be analyzed and quantified, and the exosome with CXCR4 surface markers can be measured and quantified to define the proportion of the CXCR4 surface markers that the therapeutic exosome should possess.

### 3.2 Intravenous injection of the exosome of the present disclosure augments the endogenous Nestin-GFP⁺ cell-induced neurogenesis to the peri-infarct area

To gain further insight into the regenerative potential of endogenous Nestin-GFP⁺ cells after each treatment protocol, Nestin-*GFP* gene transgenic mice were used to construct MCAO mice (hereinafter referred to as GFP mice) to investigate Nestin-GFP⁺ cells migration and proliferation into the sensorimotor cortex post-stroke. GFP mice were divided into five groups, four of which were infected with LV-sh-FOXO3 or LV-sh-control 7 days after the stroke, and then administered 100-200 µg of EXO or EXO-PD-L1-HGF to each GFP mouse (approximately 25 g) for treatment. The other group was injected with PBS as a control group. The experiment also included a sham group of mice and analyzed by the immunofluorescence staining. The mice in the sham group underwent the same surgical steps as the GFP mice, but the left anterior descending coronary artery of the mice in the sham group was not blocked.

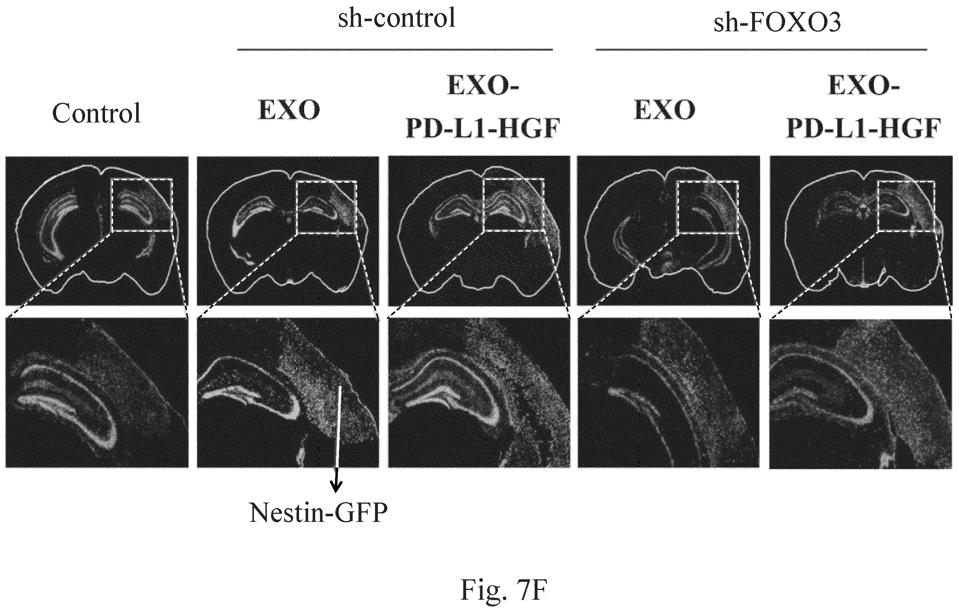

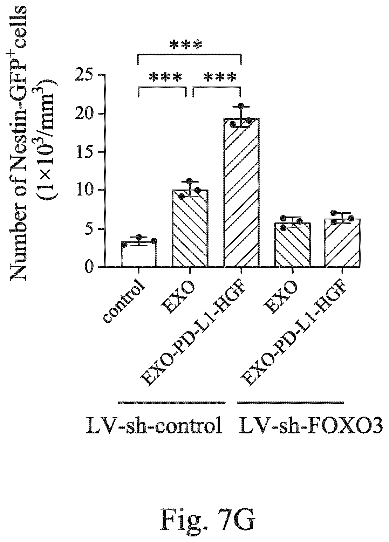

Reference is made to Fig. 7F and Fig. 7G, which are results of the immunofluorescence staining showing that the knockdown FOXO3 expression suppresses the EXO-PD-L1-HGF promoted Nestin-GFP expressed NPCs migrating to the injury site. The results show that in the groups without attenuated FOXO3 expression, significant increase in numbers of Nestin-GFP⁺ cells were migrated over the lateral and ventral peri-infarct areas in the EXO-PD-L1-HGF group compared to that of the EXO group and the control group. On the contrary, knockdown FOXO3 expression repressed the EXO-PD-L1-HGF-promoted Nestin-GFP⁺ cells migration in infarct areas.

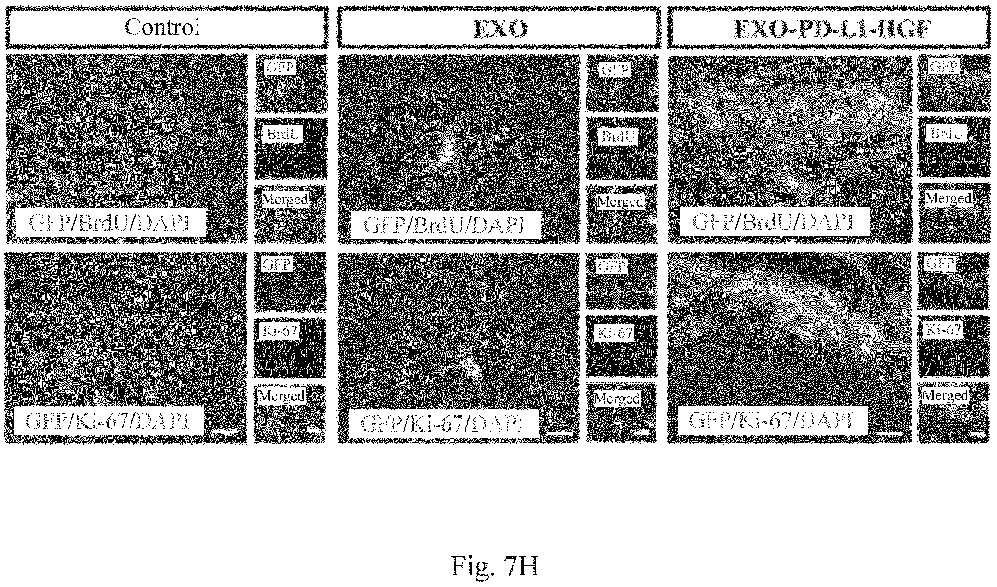

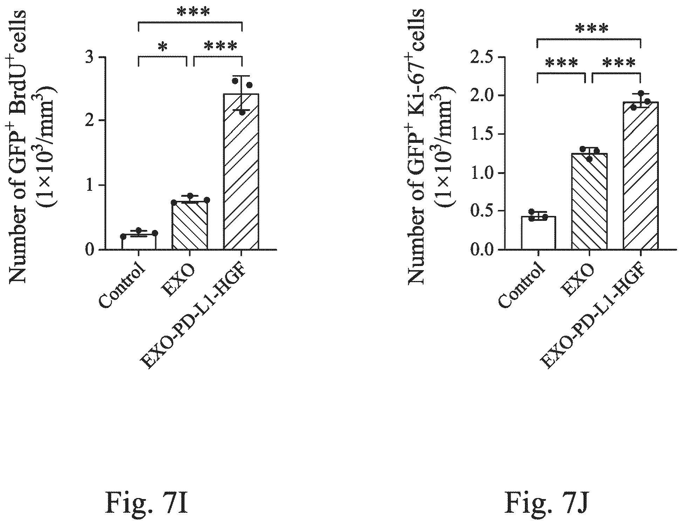

In addition, bromodeoxyuridine (BrdU, Sigma-Aldrich) and Ki-67 (Abcam) were used as cell proliferation markers to observe the proliferation of Nestin-GFP⁺ cells in the brains of GFP mice after treatment with EXO and EXO-PD-L1-HGF. Reference is made to Fig. 7H, Fig. 7I and Fig. 7J, which show analysis results of the treatment with EXO-PD-L1-HGF to induce the proliferation of Nestin-GFP expressed cells. Fig. 7I and Fig. 7J show the statistical results of Fig. 7H, and results are presented as means ± SD, wherein * represents p<0.05, and *** represents p<0.001. The results in Fig. 7H to Fig. 7J show that EXO-PD-L1-HGF administration also induced significant increase the proliferation of GFP⁺BrdU⁺ and GFP⁺Ki-67⁺ cells.

Previous study has demonstrated that the ischemic stroke can stimulated the proliferation of endogenous NPCs, which ectopically migrated to the infarct area. Moreover, functional integration of proliferated NPCs with subsequent adult neurogenesis enhanced the stroke recovery. To investigate whether the exosome of the present disclosure promoted endogenous NPCs proliferation to induce neuro-glial differentiation and then adult neurogenesis for functional recovery, examination of colocalization of neuroglial markers and proliferation markers with Nestin-GFP⁺ populations was performed by immunohistochemistry.

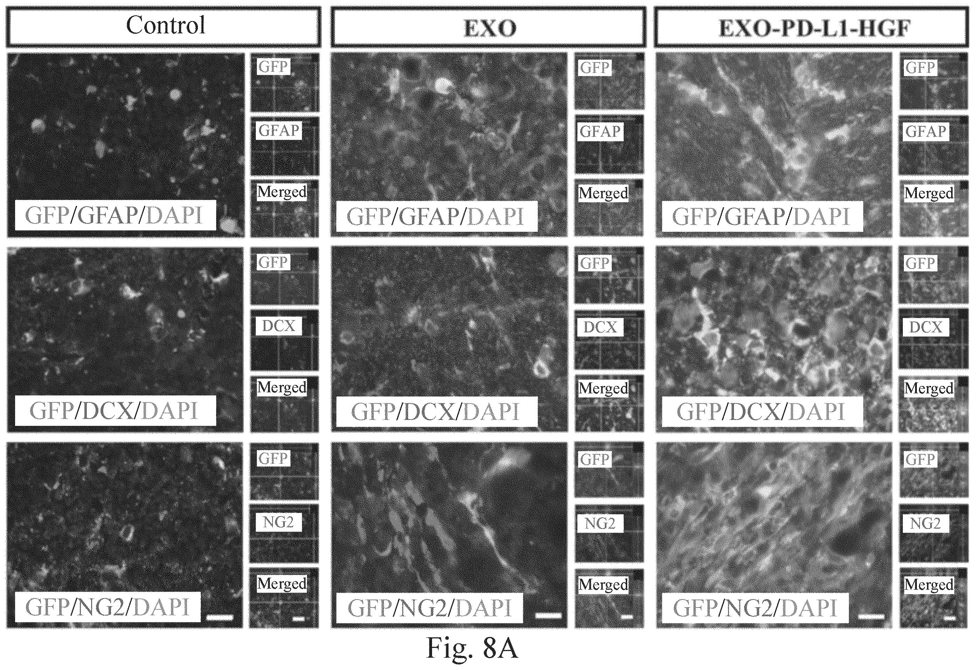

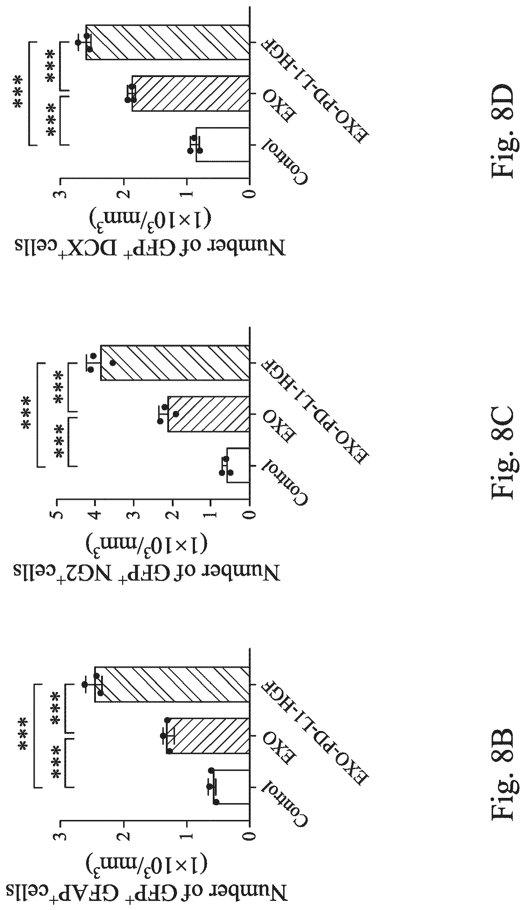

Reference is made to Fig. 8A, Fig. 8B, Fig. 8C and Fig. 8D, which show analysis results of treating EXO and EXO-PD-L1-HGF to promote the differentiation of the NPCs. Fig. 8B, Fig. 8C and Fig. 8D show the statistical results of Fig. 8A, and results are presented as means ± SD, wherein *** represents p<0.001. The results in Fig. 8A to Fig. 8D show that besides the Nestin-GFP⁺ cells expressed the oligodendrocyte (GFP⁺NG2⁺) and astrocyte (GFP⁺GFAP⁺), the immature neuron expressed with marker of doublecortin (DCX) from Nestin-GFP⁺ populations could be observed in GFP mice after the stroke. Importantly, more GFP⁺DCX⁺, GFP⁺NG2⁺ and GFP⁺GFAP⁺ cells in the peri-infarcted region at 1 week after the stroke was observed within the ventral peri-infarct region in the EXO-PD-L1-HGF group than that of the EXO group and the control group.

3.3 Activation of FOXO3 signaling modulates the exosome of the present disclosure-enhanced functional recovery and infarct volume reduction following the stroke
To verify the neuroregenerative ability of EXO-PD-L1-HGF through turning-on FOXO3 pathway in the MCAO mice, neurological functional assessments were performed on the MCAO mice in different groups, and the MCAO mice were sacrificed on 28 days after treatment. The infarct volume measurement was performed by triphenyltetrazolium chloride (TTC) staining, and the infarct volume of the MCAO mice in different groups was measured. The experimental groups included the sham group, the PBS group, the EXO group (with genotype FOXO3^{+/+}), the EXO-PD-L1-HGF group (with genotype FOXO3^{+/+}), the LV-sh-FOXO3+EXO group (with genotype FOXO3^{-/-}) and the LV-sh-FOXO3+EXO-PD-L1-HGF group (with genotype FOXO3^{-/-}).

Neurobehavioral assessments were performed in the MCAO mice on 5 days before cerebral ischemia/reperfusion model, and 0, 7, 14, 21 and 28 days after the exosome (EXO or EXO-PD-L1-HGF) treatment. The neurobehavioral assessment includes a rotarod test and a beam walking test. The baseline-test scores were recorded in order to normalize those taken after cerebral ischemia. (1) An automated rotarod treadmill consisting of a roller with 5 cm diameter suitably machined to provide grip and a power source to turn the roller was used in the rotarod test. Five circular separators divided the rod into equal-sized compartments (5 cm length each), enabling four MCAO mice to be on the treadmill simultaneously. The time spent by each of the MCAO mice on the rotarod (20-25 rpm) was measured by timers, which detected when the MCAO mice fell off the treadmill onto the plate below or remained on the rod up to 3 *minutes.* (2) The balance beam was a rod of 160 cm in length and a diameter of 2.5 cm. A plastic platform (7 cm×4 cm) was set at one end of the rod as the start, and a black plastic box (15 cm×15 cm×8 cm) was at the other end of the rod as a nest for motivating the animal crossing from the beam. The apparatus was suspended 90 cm above a cushion, which protected the fallen animals from injury, and 50 cm from a wall. The time taken the MCAO mice to traverse the beam was recorded.

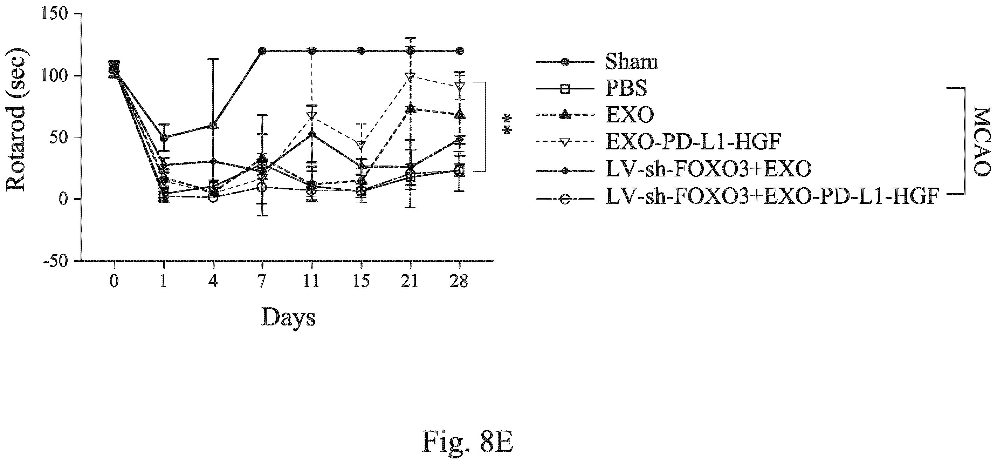

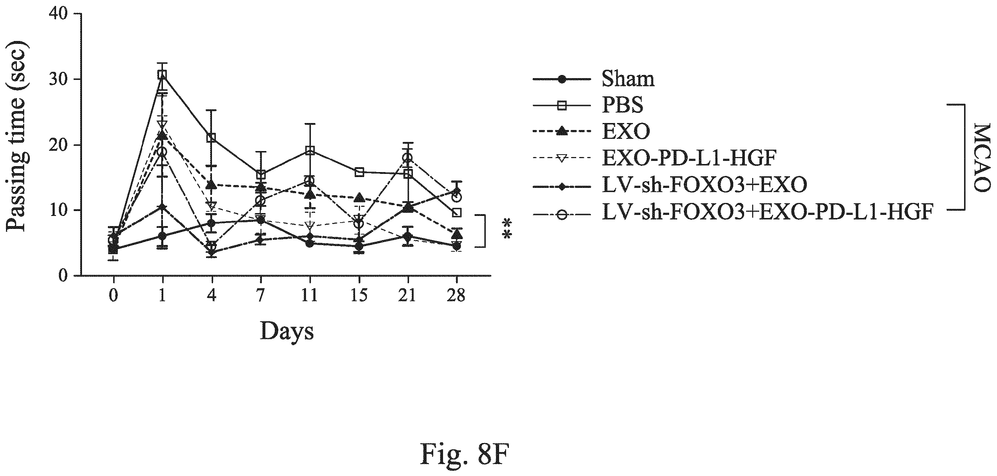

Reference is made to Fig. 8E and Fig. 8F. Fig. 8E shows analysis results of the MCAO mice treated with EXO-PD-L1-HGF in the rotarod test, and Fig. 8F shows analysis results of the MCAO mice treated with EXO-PD-L1-HGF in the beam walking test. The results in Fig. 8E and Fig. 8F show that significant improvement of neuro-behavior examination by the rotarod test and the beam walking test was demonstrated in the EXO-PD-L1-HGF group during 28 days after the ischemic stroke compared with the other groups. In contrast, the EXO-PD-L1-HGF-induced improvement on neurological function was significantly suppressed in the LV-sh-FOXO3+EXO group and the LV-sh-FOXO3+EXO-PD-L1-HGF group.

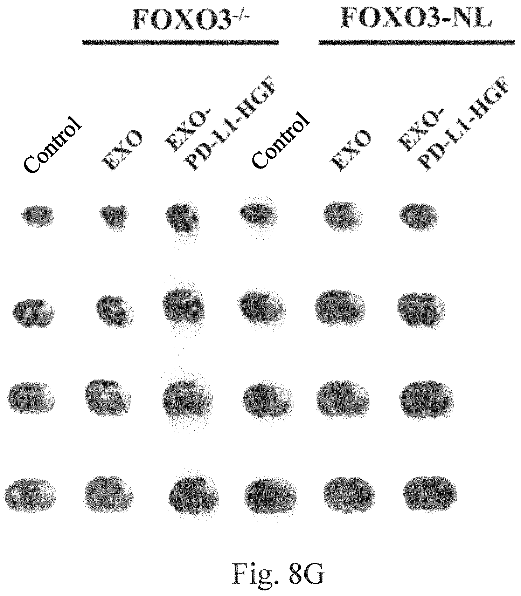

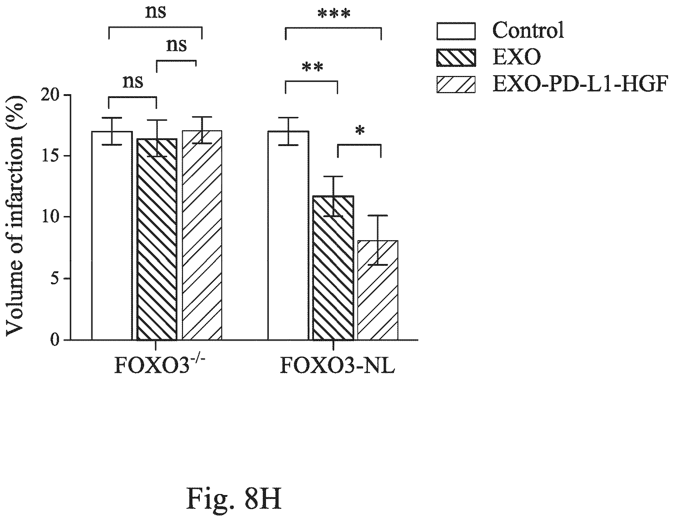

Reference is made to Fig. 8G and Fig. 8H, which show analysis results of the brain infarction in the MCAO mice treated with EXO-PD-L1-HGF. Fig. 8H shows the statistical results of Fig. 8G, and results are presented as means ± SD, wherein * represents p<0.05, ** represents p<0.01, and *** represents p<0.001. The results in Fig. 8G and Fig. 8H show that in the FOXO3-NL group with genotype FOXO3^{+/+}, the infarction volume of the ischemic cerebral hemisphere was smaller in the EXO-PD-L1-HGF group than that of the EXO group and the control group by TTC staining. However, EXO-PD-L1-HGF-induced reduction of infarction volume was not observed in the groups with genotype FOXO3^{-/-}.

The experiment also tested whether administration of UMSC-EXO-PD-L1-HGF could also improve functional recovery and reduce infarct volume in the MCAO mice after the stroke. The MCAO mice were constructed and then divided into three groups, two of which were administered 100-200 µg of UMSC-EXO or UMSC-EXO-PD-L1-HGF to each MCAO mouse (approximately 25 g) for treatment. The other group was injected with PBS as a control group. Neurobehavioral assessments including the rotarod test and the beam walking test were performed in the MCAO mice on 5 days before cerebral ischemia/reperfusion model, and 0, 7, 14, 21 and 28 days after the exosome (UMSC-EXO or UMSC-EXO-PD-L1-HGF) treatment. The MCAO mice were sacrificed on 28 days after treatment, and the infarct volume of each of the MCAO mice was measured by TTC staining.

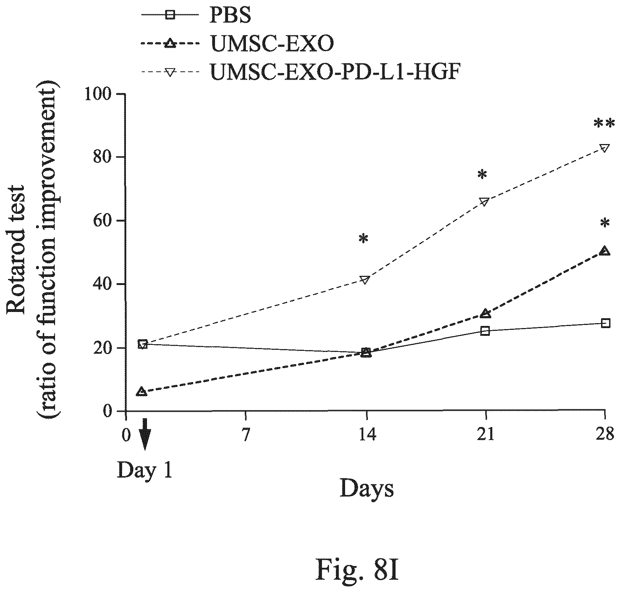

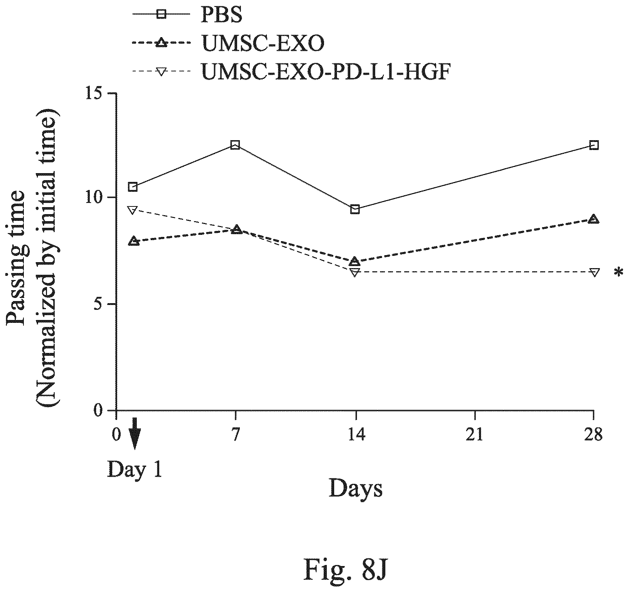

Reference is made to Fig. 8I and Fig. 8J. Fig. 8I shows analysis results of the MCAO mice treated with UMSC-EXO-PD-L1-HGF in the rotarod test, and Fig. 8J shows analysis results of the MCAO mice treated with UMSC-EXO-PD-L1-HGF in the beam walking test. The results show that significant improvement of neuro-behavior examination by the rotarod test and the beam walking test was demonstrated in the UMSC-EXO-PD-L1-HGF group on 28 days after the ischemic stroke compared with the other groups. The above results indicate that the exosome of the present disclosure can significantly improve the neurological behavior of the MCAO mice and reduce the infarct volume of the MCAO mice.

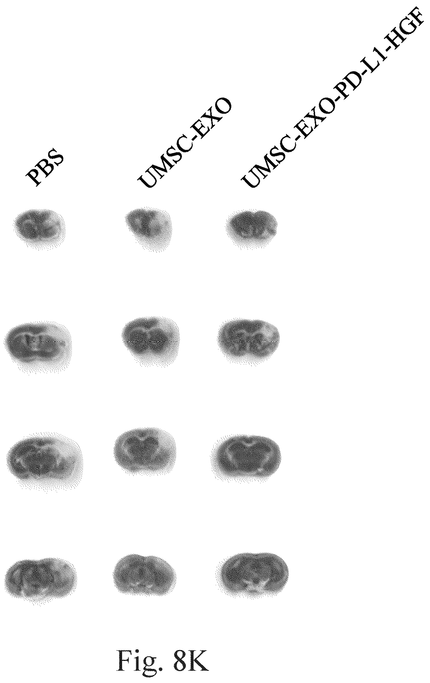

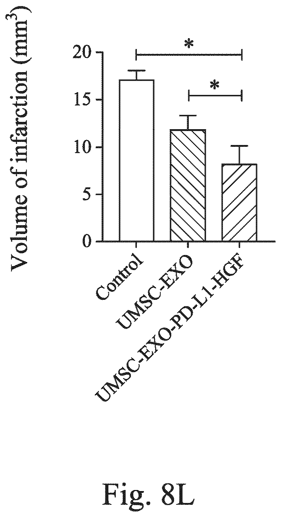

Reference is made to Fig. 8K and Fig. 8L, which show analysis results of the brain infarction in the MCAO mice treated with UMSC-EXO-PD-L1-HGF. Fig. 8L shows the statistical results of Fig. 8K, and results are presented as means ± SD, wherein * represents p<0.05. The TTC staining results showed that the infarction volume of ischemic cerebral hemisphere was smaller in the UMSC-EXO-PD-L1-HGF group than that of the UMSC-EXO group and the control group. The above results show that the exosome of the present disclosure can regulate neuroplasticity in the stroke brain.

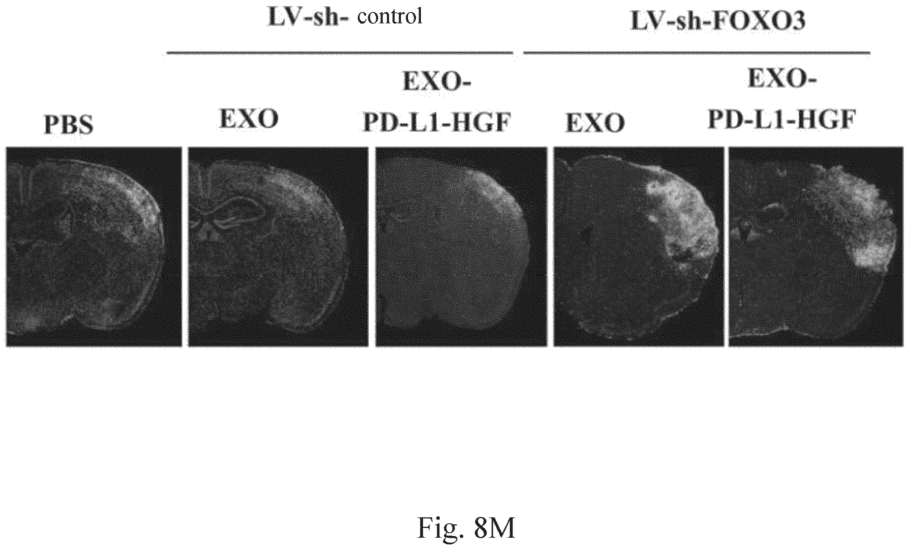

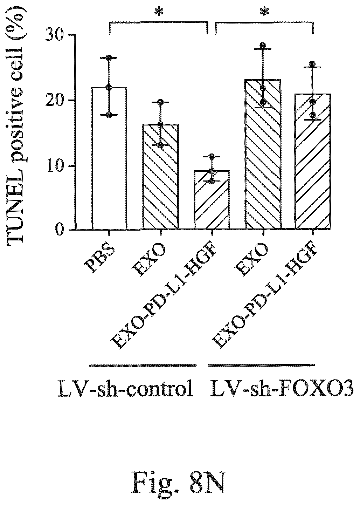

Furthermore, cell apoptosis in the brain tissues of the MCAO mice treated with EXO-PD-L1-HGF was detected by the TUNEL staining. Reference is made to Fig. 8M and Fig. 8N, which show analysis results of the TUNEL staining, wherein Fig. 8N shows the statistical results of Fig. 8M, and results are presented as means ± SD, wherein * represents p<0.05. The results show that the number of TUNEL positive cells of ischemic cerebral hemisphere treated with the EXO-PD-L1-HGF group was significantly reduced, whereas the LV-sh-FOXO3 treatment abolished the EXO-PD-L1-HGF-induced diminishment of TUNEL positive cells in the ischemic cerebral hemisphere. In summary, the results suggest that FOXO3 activation switched on the EXO-PD-L1-HGF-modulated neuroplasticity in the stroke brain.

### 3.4 The exosome of the present disclosure can control immune regulation

To demonstrate whether the exosome of the present disclosure could manipulate the immunoregulation, percentage of leukocytes population in the brain and the spleen of the MCAO mice or the mice in the sham group treated with EXO and EXO-PD-L1-HGF were examined by the flow cytometry respectively.

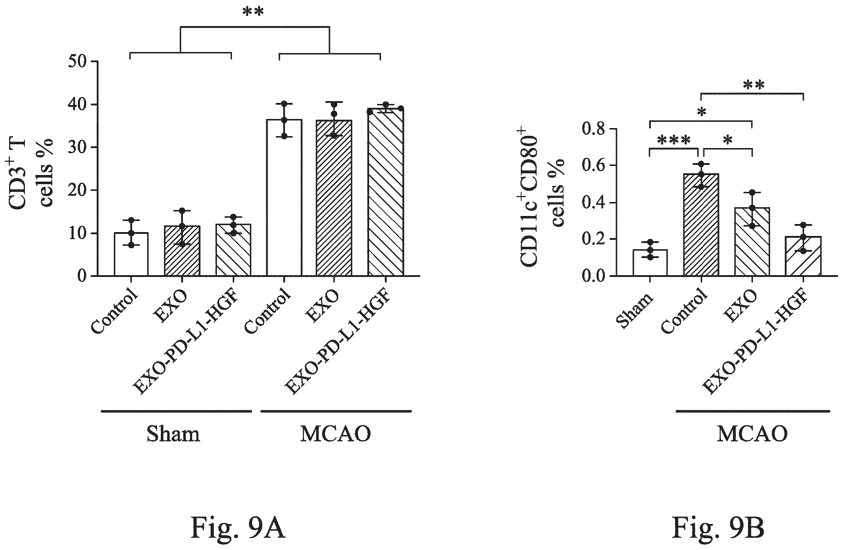

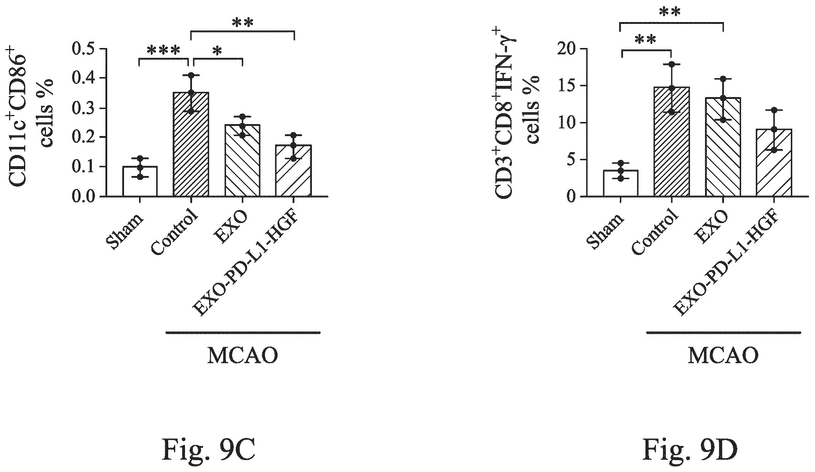

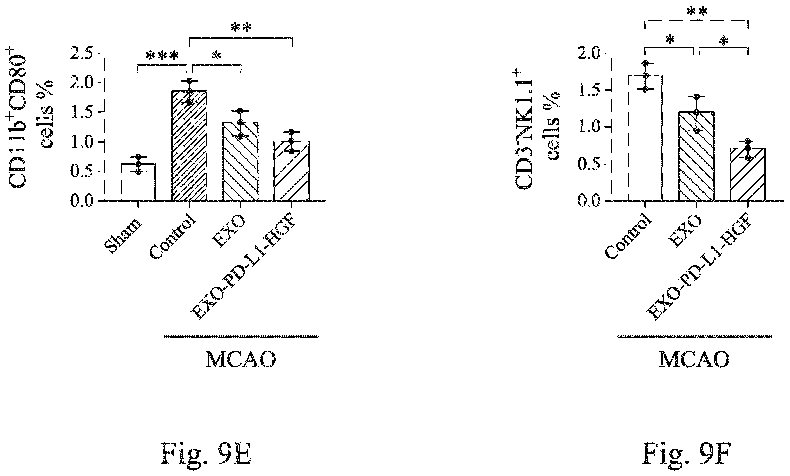

Reference is made to Fig. 9A, which shows analysis results of the number of CD3⁺ T cells in the brain tissues of mice in different groups. The results show that the ischemic cerebral hemisphere treated with EXO-PD-L1-HGF revealed a significant increase in the total number of viable leukocytes including CD3⁺ T cells compared with the sham group, while the total cell numbers did not change between the two cerebral hemispheres in the control group, the EXO group and the EXO-PD-L1-HGF group.

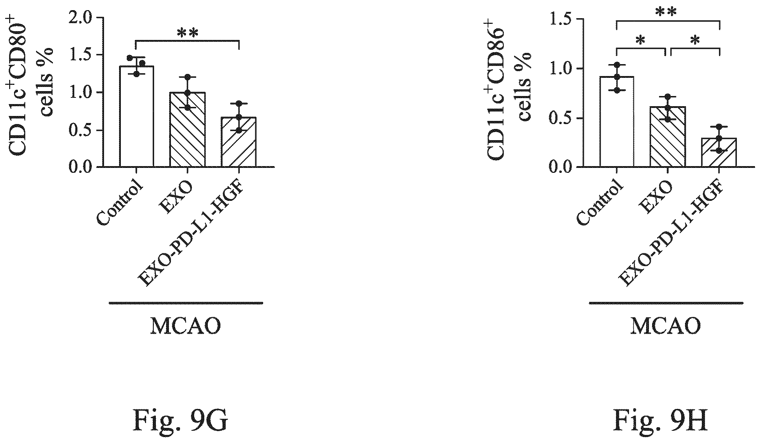

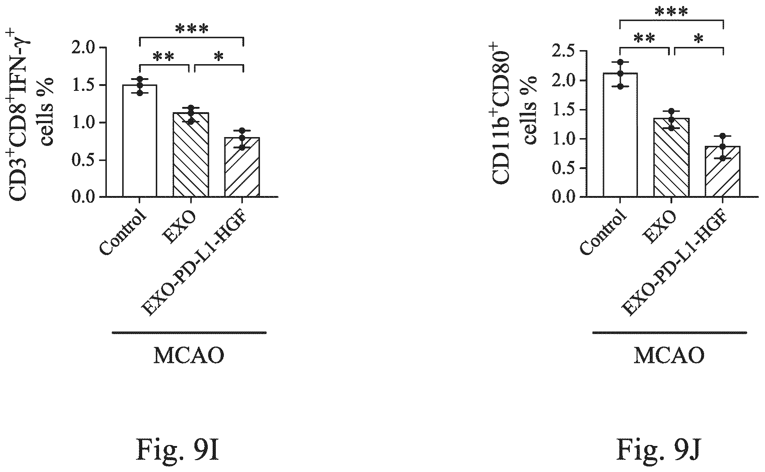

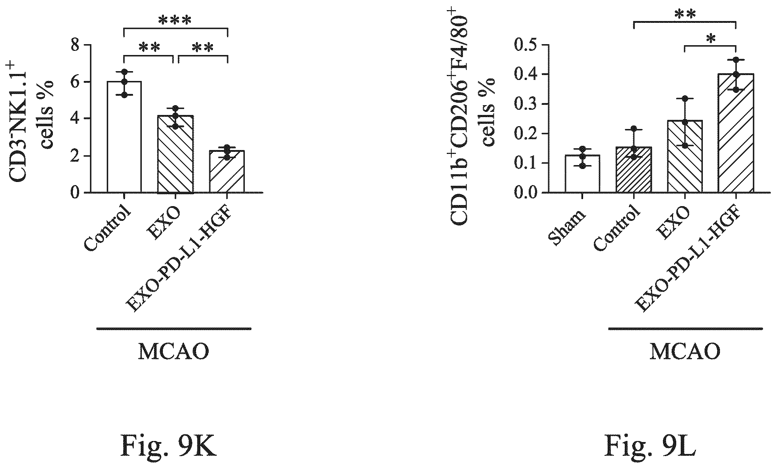

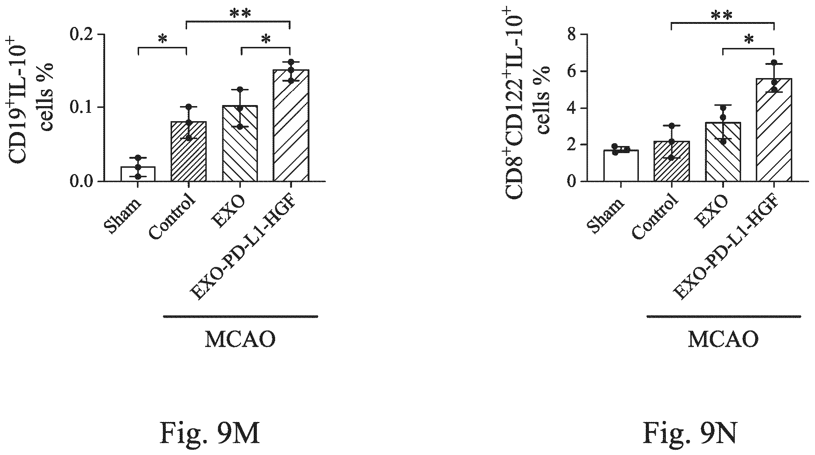

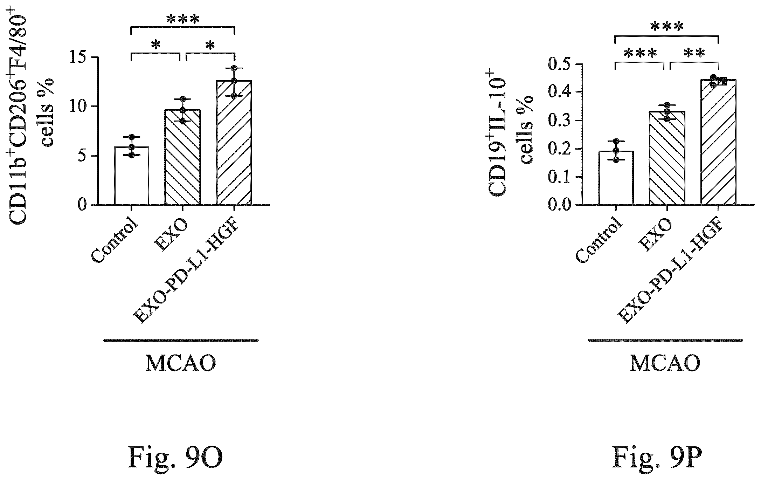

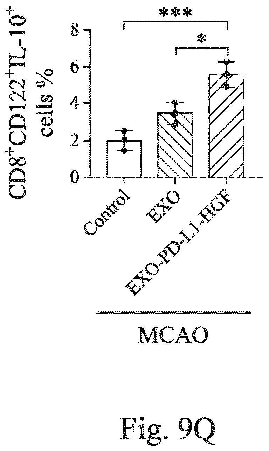

Reference is made to Fig. 9B to Fig. 9Q, Fig. 9B, Fig. 9C, Fig. 9D, Fig. 9E and Fig. 9F show analysis results of repressing the percentage of activated dendritic cells (DCs), cytotoxic T cells, natural killer (NK) cells and M1 macrophages in the ischemic cerebral hemisphere after the treatment with EXO or EXO-PD-L1-HGF; Fig. 9G, Fig. 9H, Fig. 9I, Fig. 9J and Fig. 9K show analysis results of repressing the percentage of activated DCs, cytotoxic T cells, NK cells and M1 macrophages in the spleen after the treatment with EXO or EXO-PD-L1-HGF; Fig. 9L, Fig. 9M and Fig. 9N show analysis results of enhancing the percentage of regulatory B cells, regulatory T cells and M2 macrophages in the ischemic cerebral hemisphere after the treatment with EXO or EXO-PD-L1-HGF; Fig. 9O, Fig. 9P and Fig. 9Q show analysis results of enhancing the percentage of regulatory B cells, regulatory T cells and M2 macrophages in the spleen after the treatment with EXO or EXO-PD-L1-HGF.

The results in Fig. 9B to Fig. 9K show that administration of EXO-PD-L1-HGF by intravenous injection in the MCAO mice significantly reduced the percentage of activated DC cells (CD11c⁺CD80⁺ and CD11c⁺CD86⁺), cytotoxic T cells (CD3⁺CD8⁺IFN-γ⁺), NK cells (CD3⁻NK1.1⁺), M1 macrophage (CD11b⁺CD80⁺) in comparison to that of the EXO group and the control group in the ischemic cerebral hemisphere and the spleen. The results in Fig. 9L to Fig. 9Q show that administration of EXO-PD-L1-HGF by intravenous injection in the MCAO mice significantly enhanced the percentages of regulatory B cells (CD19⁺IL-10⁺), regulatory T cells (CD8⁺CD122⁺IL-10⁺) and M2 macrophage (CD11b⁺CD206⁺F4/80⁺) in comparison to that of the EXO group and the control group in the ischemic cerebral hemisphere and the spleen. The above results show that the exosome of the present disclosure can reduce pro-inflammatory responses by regulating anti-inflammatory leukocytes in the ischemic brain and the spleen.

In summary, the exosome of the present disclosure can efficiently be endocytosed into target cells, significantly diminish the H₂O₂-induced neurotoxicity, and increase the expressions of the anti-apoptotic proteins in neurons through induction of some cytokines. The exosome of the present disclosure can attenuate inflammatory damage by inhibiting the proliferation of T cell and reducing IFN-γ⁺cells. The exosome of the present disclosure also can facilitate neurogenesis and angiogenesis induced by endogenous progenitor cells via STAT3-FOXO3 signaling axis, which plays a pivotal role in cell survival and neuroprotection to induce remarkable mitigation of infarction volume and enhancement of neurological recovery in the ischemic stroke mouse model. Moreover, the exosome of the present disclosure can provide an anti-inflammatory microenvironment to protect neurons in the ischemic brain by increasing the number of regulatory B cells and regulatory T cells and reducing the number of DCs, NK cells, and cytotoxic T cells. Therefore, the exosome of the present disclosure can be used as a new drug delivery strategy and can strikingly modulate a niche for inducing neuroplastic regeneration in the stroke brain. The data in the specification indicate that the exosome of the present disclosure and pharmaceutical composition including the exosome of the present disclosure are not only safe and effective, but also can play an important role in rescuing brain ischemic damage.

## Claims

1. An exosome derived from a genetically engineered mesenchymal stem cell, wherein the genetically engineered mesenchymal stem cell comprises an exogenous *PD-L1* gene and an exogenous *HGF* gene.

2. The exosome of claim 1, wherein the exogenous *PD-L1* gene comprises the amino acid sequence of SEQ ID NO: 4, and the exogenous *HGF* gene comprises the amino acid sequence of SEQ ID NO: 5.

3. The exosome of claims 1 or 2, wherein the exogenous *PD-L1* gene and the exogenous *HGF* gene are linked by a self-cleaving peptide coding fragment.

4. The exosome of any one of claims 1 to 3, wherein the exosome comprises an overexpression PD-L1 and an overexpression HGF.

5. The exosome of claim 4, wherein when measured by a flow cytometry, a PD-L1 expression level on an exosome membrane of the exosome is increased relative to a control exosome, and the control exosome is derived from a non-genetically engineered mesenchymal stem cell.

6. The exosome of claim 4, wherein when measured by an ELISA, a HGF content of the exosome is increased relative to a control exosome, and the control exosome is derived from a non-genetically engineered mesenchymal stem cell.

7. The exosome of any one of claims 1 to 6, wherein the exosome comprises an overexpression CXCR4 on an exosome membrane thereof.

8. The exosome of claim 7, wherein when measured by a flow cytometry, a proportion of the exosome with CXCR4 surface marker is increased relative to a control exosome, and the control exosome is derived from a non-genetically engineered mesenchymal stem cell.

9. The exosome of any one of claims 1 to 8, wherein a particle size of the exosome ranges from 30 nm to 200 nm.

10. The exosome of claim 9, wherein the particle size of the exosome ranges from 100 nm to 150 nm.

11. A preparation method of an exosome, **characterized in** comprising:
constructing a genetically engineered mesenchymal stem cell, wherein an exogenous *HGF* gene and an exogenous *PD-L1* gene are transferred into a mesenchymal stem cell to obtain the genetically engineered mesenchymal stem cell;
performing a culture step, wherein the genetically engineered mesenchymal stem cell is cultured in a culture medium to obtain a conditioned medium; and
performing an isolation step, wherein an exosome is collected from the conditioned medium by an isolating method.

12. The preparation method of the exosome of claim 11, wherein in the culture step, the genetically engineered mesenchymal stem cell is cultured under a hypoxic condition.

13. The preparation method of the exosome of claim 12, wherein the hypoxic condition is an oxygen content of 3% or less.

14. The preparation method of the exosome of any one of claims 11 to 13, wherein the mesenchymal stem cell is an adipose mesenchymal stem cell, an umbilical cord mesenchymal stem cell or a bone marrow mesenchymal stem cell.

15. The preparation method of the exosome of any one of claims 11 to 14, wherein the mesenchymal stem cell is an adipose mesenchymal stem cell or an umbilical cord mesenchymal stem cell.

16. A pharmaceutical composition for treating an ischemia condition of a tissue, **characterized in** comprising:
the exosome of claim 1; and
a pharmaceutically acceptable carrier.

17. The pharmaceutical composition of claim 16, wherein an administration route of the pharmaceutical composition is an intravenous injection, an intracarotid artery injection, an intraarterial injection or a combination thereof.

18. The exosome of claim 1 for use in treatment of an ischemia condition of a tissue.

19. The exosome for use of claim 18, wherein the tissue is a brain.

20. The exosome for use of claim 19, wherein the ischemia condition is an ischemic stroke.

21. The exosome for use of claim 20, wherein the exosome reduces an area of the brain damaged by the ischemic stroke.

22. The exosome of claim 1 for use in enhancing neuroregeneration or reducing neuron death.

23. The exosome of claim 1 for use in reducing an inflammatory response.
